# EUROPEAN PATENT SPECIFICATION

(11) **EP 4 580 599 B1**
(45) Date of publication and mention of the grant of the patent: **28.10.2026**
(21) Application number: 23764280.6
(22) Date of filing: 29.08.2023
(51) Int. Cl.: A61K 9/00, A61K 9/107, A61K 47/44, A61P 3/10, A61P 43/00

(54) **LIPID EMULSION WITH ANTI-INFLAMMATORY EFFECTS FOR TOTAL PARENTERAL AND ENTERAL NUTRITION**
LIPIDEMULSION MIT ENTZÜNDUNGSHEMMENDER WIRKUNG FÜR TOTALE PARENTERALE UND ENTERALE ERNÄHRUNG
EMULSION LIPIDIQUE A EFFETS ANTI-INFLAMMATOIRES POUR NUTRITION ENTERALE ET PARENTERALE TOTALE

(30) Priority: 01.09.2022 EP 22193442
(43) Date of publication of application: 09.07.2025
(73) Proprietor: Universität Zürich, 8006 Zürich (CH); ETH Zurich, 8092 Zürich (CH); University of Alberta, Edmonton AB T6G 2G3 (CA)
(72) Inventor: HERSBERGER, Martin, 8006 Zürich (CH); ROGLER, Gerhard, 8006 Zürich (CH); KRÄMER, Stefanie, 8092 Zürich (CH); HOLTZHAUER, Gregory, 8092 Zürich (CH); ZAUGG, Michael, Edmonton, AB T6G 2G3 (CA); LUCCHINETTI, Eliana, Edmonton, AB T6G 2G3 (CA)
(74) Representative: Schulz Junghans Patentanwälte PartGmbB
(86) International application number: PCT/EP2023/073737
(87) International publication number: WO 2024/047075

(56) References cited:
- KR-A- 20180 004 096
- US-A1- 2008 039 525
- KEVIN A HARVEY ET AL: "Modulation of endothelial cell integrity and inflammatory activation by commercial lipid emulsions", LIPIDS IN HEALTH AND DISEASE, BIOMED CENTRAL, LONDON, GB, vol. 14, no. 1, 18 February 2015 (2015-02-18), pages 9, XP021211646, ISSN: 1476-511X, DOI: 10.1186/S12944-015-0005-6

## Description

This application claims the right of priority of European Patent Application EP22193442.5 filed 01 September 2022,

### Field

The present invention relates to lipid emulsions for total parenteral and enteral (oral or through gastric or duodenal tubes) nutrition and administration of pharmaceutical drugs. The lipid emulsions according to the invention have advantageous immuno-modulating, anti-inflammatory, and anti-diabetic effects. The lipid emulsions may comprise pharmaceutical drugs, and can be used as detoxifiers or to reverse negative effects arising from ischemia-reperfusion injury.

### Background of the Invention

Parenteral lipid emulsions (LE) are heterogeneous systems, consisting of an oily phase homogeneously dispersed in an aqueous phase in the presence of an emulsifier. A droplet size, usually between 200 and 350 nm, characterizes these lipid emulsions suitable for parenteral administration and they have a physiological pH around 7, isotonicity, and a high zeta potential, to prevent instability. The currently available commercial lipid emulsions consist of triglycerides from plant or fish oils such as soybean oil, olive oil, coconut oil, fish oil, and others or blends thereof (Table 1), egg yolk lecithin (emulsifier), glycerol (to provide isotonicity), and water. Lipid emulsions are used in clinical nutrition, both for parenteral and enteral administration, and their fatty acid composition can influence metabolic and immune responses. For example, [XP021211646 (2015-02-18)] describes the effects of various commercial lipid emulsions, including those based on soybean oil, olive oil, and fish oil, on endothelial cell integrity and inflammatory activation. These emulsions contain different proportions of omega-3, omega-6, omega-9, and saturated fatty acids, and their cellular uptake and biological effects have been investigated. Other approaches have focused on optimizing the ratios of omega-3, omega-6, and omega-9 fatty acids in nutritional products, with the aim of supporting glucose metabolism and vascular health (see [US2008039525A1 (2008-02-14)]). Some nutritional compositions have also been developed for food, topical, or parenteral use, with attention to balancing essential fatty acids and including monounsaturated and saturated fatty acids (see [KR20180004096A (2018-01-10)]). However, there remains a need for lipid emulsions with specifically tailored fatty acid profiles, including defined levels of stearidonic acid (SDA) and alpha-linolenic acid (ALA), as well as precise ranges of omega-6, monounsaturated, and saturated fatty acids.

Based on the above-mentioned state of the art, the objective of the present invention is to provide an immuno-modulating, anti-inflammatory, and anti-diabetic lipid emulsion for total parenteral nutrition and enteral nutrition and administration. This objective is attained by the subject-matter of the independent claims of the present specification, with further advantageous embodiments described in the dependent claims, examples, figures and general description of this specification.

### Summary of the Invention

A first aspect of the invention relates to a lipid emulsion for parenteral administration, wherein the lipid emulsion comprises an oily phase and an aqueous phase, wherein the oily phase of the lipid emulsion comprises:
- an omega-3 fatty acid component,
- an omega-6 fatty acid component,
- a monounsaturated fatty acid component, and
- a saturated fatty acid component

in the ratios and relationships given in the claims, the detailed description of the invention, and the examples (any % values given throughout this document to be interpreted as mass/mass unless stated otherwise).

A second aspect of the invention relates to a lipid emulsion according to the first aspect and its embodiments for use in parenteral or enteral nutrition.

A third aspect of the invention relates to a lipid emulsion according to the first aspect and its embodiments for use as a counteragent/detoxifyer ("lipid sink") in a treatment of intoxication caused by a lipophilic drug.

A fourth aspect of the invention relates to a lipid emulsion according to the first aspect and its embodiments for use in protection against ischemia-reperfusion injury of vital organs. In certain embodiments, the vital organ is selected from heart, brain, liver, kidneys, and lungs.

A fifth aspect of the invention relates to a lipid emulsion according to the first aspect for use in prevention or treatment of diabetes mellitus type I and II.

### Terms and definitions

For purposes of interpreting this specification, the following definitions will apply and whenever appropriate, terms used in the singular will also include the plural and vice versa. In the event that any definition set forth below conflicts with any document the definition set forth shall control.

The terms "comprising", "having", "containing", and "including", and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *TPN* in the context of the present specification relates to total parenteral nutrition.

The term *SDA* in the context of the present specification relates to stearidonic acid.

The term *ALA* in the context of the present specification relates to α-linolenic acid.

The term F3 (see specifically attached figures) stands for Formula#3 and is used for the newly created lipid emulsion with unique physicochemical and biological effects as disclosed herein. In the specification, TPN-F3, F3, W-TPN, and VV are used synonymously.

As used herein, the term *pharmaceutical drug* refers to is a chemical substance which, when administered to a living organism, produces a biological effect. A pharmaceutical drug is a chemical substance used to treat, cure, prevent, or diagnose a disease or to promote well-being.

As used herein, the term *toxic compound* refers to a chemical substance which can damage a patient's well-being, or may even be life-threatening.

As used herein, the term *lipophilic* refers to the ability of a chemical compound to dissolve in fats, oils, lipids, and non-polar solvents.

As used herein, the term *detoxifyer* refers to the ability of a chemical substance or mixture to decrease the damage of a drug or compound to the patient's body.

As used herein, the term *ischemia-reperfusion injury* refers to the tissue damage caused when blood supply returns to tissue after a period of ischemia or lack of oxygen (anoxia or hypoxia).

As used herein, the term *pharmaceutical composition* refers to an emulsion of the invention, or a pharmaceutically acceptable salt thereof, together with at least one pharmaceutically acceptable carrier. In certain embodiments, the pharmaceutical composition according to the invention is provided in a form suitable for parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. diabetes) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

A first aspect of the invention relates to a lipid emulsion for parenteral administration, wherein the lipid emulsion comprises an oily phase and an aqueous phase, wherein the oily phase of the lipid emulsion comprises (all % values as mass/mass):
- an omega-3 fatty acid component,
- an omega-6 fatty acid component,
- a monounsaturated fatty acid component, and
- a saturated fatty acid component.

The mass of the omega-3 fatty acid component amounts to 20-50 %, the mass of the omega-6 fatty acid component amounts to 3-35 %, the mass of the monounsaturated fatty acid component amounts to 5-40 %, and the mass of the saturated fatty acid component amounts to 5-45 % of the oily phase.

In certain embodiments, the mass of the omega-3 fatty acid component amounts to 20-40 %, the mass of the omega-6 fatty acid component amounts to 5-25 %, the mass of the monounsaturated fatty acid component amounts to 10-35 %, and the mass of the saturated fatty acid component amounts to 10-40 % of the oily phase.

In certain embodiments, the mass of the omega-3 fatty acid component amounts to 25-35 %, the mass of the omega-6 fatty acid component amounts to 10-15 %, the mass of the monounsaturated fatty acid component amounts to 18-30 %, and the mass of the saturated fatty acid component amounts to 20-35 % of the oily phase.

In certain embodiments, the mass of the omega-3 fatty acid component amounts to ~32 %, the mass of the omega-6 fatty acid component amounts to ~12 %, the mass of the monounsaturated fatty acid component amounts to ~27 %, and the mass of the saturated fatty acid component amounts to ~29 % of the oily phase.

The omega-3 fatty acid component consists of one or more omega-3 (C₁₀-C₂₄ alkyl-oligo-ene carboxylic acids) fatty acids characterized by the presence of more than one carbon double bonds, wherein one carbon-carbon *cis* double bond is three atoms away from the terminal methyl group (exemplary structure of the omega-3 fatty acid α-linolenic acid (C18:3 omega-3);

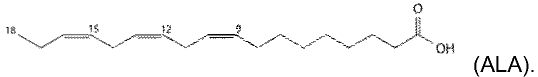

In certain embodiments, the omega-3 fatty acid component consists of one or several of the members of the group comprised of α-linolenic acid and stearidonic acid.

The oily phase of the lipid emulsion comprises ≥ 5 % stearidonic acid (C18:4 omega-3) as part of the omega-3 fatty acid component.

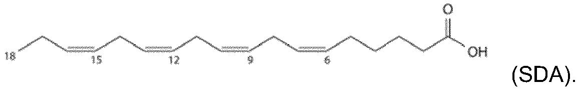

In certain embodiments, the oily phase of the lipid emulsion comprises ~ 10 % stearidonic acid as part of the omega-3 fatty acid component.

The oily phase of the lipid emulsion comprises ≥ 15 % α-linolenic acid (ALA) (C18:3 omega-3) as part of the omega-3 fatty acid component.

In certain embodiments, the oily phase of the lipid emulsion comprises ~ 20 % α-linolenic acid as part of the omega-3 fatty acid component.

The omega-6 fatty acid component consists of one or more (C₁₀-C₂₄ alkyl-oligo-ene carboxylic acids) omega-6 fatty acids characterized by the presence of more than one carbon double bonds, wherein one carbon-carbon *cis* double bond six atoms away from the terminal methyl group (exemplary structure of the omega-6 fatty acid linoleic acid (C18:2 omega-6));

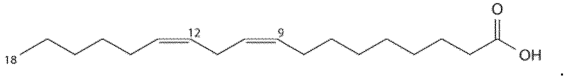

In certain embodiments, the omega-6 fatty acid component consists of one or several of the members of the group comprised of: linoleic acid and γ-linolenic acid.

The monounsaturated fatty acid component consists of one or more fatty acids characterized by the presence of one carbon-carbon double bond.

In certain embodiments, the monounsaturated fatty acid component comprises or consists of oleic acid (CAS No 112-80-1).

The saturated fatty acid component consists of one or more fatty acid characterized by no carbon-carbon double bond, but only carbon-carbon single bonds.

In certain embodiments, the saturated fatty acid component comprises or consists of one or several of the members of the group comprised of: caproic acid, caprylic acid, capric acid, lauric acid, myristic acid, and palmitic acid.

In certain embodiments, the ratio (m/m) of the omega-6 fatty acid component to the omega-3 fatty acid component is 1:5 to 2:1. In certain embodiments, the ratio (m/m) of the omega-6 fatty acid component to the omega-3 fatty acid component is 1:4 to 1:1. In certain embodiments, the ratio (m/m) of the omega-6 fatty acid component to the omega-3 fatty acid component is 1:3 to 1:2. In certain embodiments, the ratio (m/m) of the omega-6 fatty acid component to the omega-3 fatty acid component is ~1:2.6.

In certain embodiments, the oily phase of the lipid emulsion comprises:
- 30-60 % PUFA (polyunsaturated fatty acid);
- 5-45 % MUFA (monounsaturated fatty acid); and
- 5-50 % SFA (saturated fatty acid).

In certain embodiments, the oily phase of the lipid emulsion comprises:
- 35-55 % PUFA;
- 10-40 % MUFA; and
- 10-45 % SFA.

In certain embodiments, the oily phase of the lipid emulsion comprises:
- 40-50 % PUFA;
- 20-30 % MUFA; and
- 20-35 % SFA.

In certain embodiments, the oily phase of the lipid emulsion comprises:
- ~44 % PUFA;
- ~27 % MUFA; and
- ~29 % SFA.

In certain embodiments, the oily phase of the lipid emulsion comprises:
- 5-35 % stearidonic acid (C18:4 omega-3);
- 5-50 % oleic acid (C18:1);
- 2-30 % linoleic acid (C18:2 omega-6);
- 5-50 % α-linolenic acid (C18:3 omega-3); and
- 0.5-15 % γ-linolenic acid (C18:3 omega-6).

In certain embodiments, the oily phase of the lipid emulsion comprises:
- 5-25 % stearidonic acid;
- 10-40 % oleic acid;
- 4-20 % linoleic acid;
- 10-40 % α-linolenic acid; and
- 1-10 % γ-linolenic acid.

In certain embodiments, the oily phase of the lipid emulsion comprises:
- 5-15 % stearidonic acid;
- 20-30 % oleic acid;
- 5-15 % linoleic acid;
- 20-30 % α-linolenic acid; and
- 2-5 % γ-linolenic acid.

In certain embodiments, the oily phase of the lipid emulsion comprises:
- ~ 10 % stearidonic acid;
- ~24 % oleic acid;
- ~9 % linoleic acid;
- ~22 % α-linolenic acid; and
- ~3 % γ-linolenic acid.

In certain embodiments, the oily phase of the lipid emulsion comprises:
- 0.5-15 % of a short-chain fatty acid component selected from caproic, caprylic, and capric acid (C6:0, C8:0, C10:0);
- 3-35 % lauric acid (C12:0);
- 1-15 % myristic acid (C14:0); and
- 1-20 % palmitic acid (C16:0).

In certain embodiments, the oily phase of the lipid emulsion comprises:
- 1-10 % of a short-chain fatty acid component selected from caproic, caprylic, and capric acid;
- 5-25 % lauric acid;
- 2-12 % myristic acid; and
- 3-15 % palmitic acid.

In certain embodiments, the oily phase of the lipid emulsion comprises:
- 2-5 % of a short-chain fatty acid component selected from caproic, caprylic, and capric acid;
- 10-15 % lauric acid;
- 3-8 % myristic acid; and
- 5-12 % palmitic acid.

In certain embodiments, the oily phase of the lipid emulsion comprises:
- ~3.5 % of a short-chain fatty acid component selected from caproic, caprylic, and capric acid;
- ~12 % lauric acid;
- ~4.5 % myristic acid; and
- ~7.9 % palmitic acid.

In certain embodiments, the oily phase of the lipid emulsion comprises:
- 8-50 % olive oil;
- 8-50 % coconut oil; and
- 20-90 *% Buglossoides arvensis* oil (Ahiflower^{®}).

In certain embodiments, the oily phase of the lipid emulsion comprises:
- 12-40 % olive oil;
- 12-40 % coconut oil; and
- 30-70 *% Buglossoides arvensis* oil (Ahiflower^{®}).

In certain embodiments, the oily phase of the lipid emulsion comprises:
- 20-30 % olive oil;
- 20-30 % coconut oil; and
- 40-60 *% Buglossoides arvensis* oil (Ahiflower^{®}).

In certain embodiments, the oily phase of the lipid emulsion comprises:
- ~25 % olive oil;
- ~25 % coconut oil; and
- ~50 *% Buglossoides arvensis* oil (Ahiflower^{®}).

In certain embodiments, the lipid emulsion additionally comprises a stabilizer and/or an anti-oxidant. In certain embodiments, the lipid emulsion additionally comprises a stabilizer and/or an anti-oxidant selected from
- **EDTA;** and/or
- alpha tocopherol.

In certain embodiments, the lipid emulsion additionally comprises a stabilizer and/or an anti-oxidant selected from
- ~2.5 µmol/L EDTA; and/or
- ~200 mg/L alpha tocopherol.

In certain embodiments, the lipid emulsion comprises
- egg yolk lecithin;
- glycerol; and
- water.

In certain embodiments, the ratio (V/V) between oily phase and aqueous phase ranges between 0.1 to 0.9. In certain embodiments, the ratio (V/V) between oily phase and aqueous phase ranges between 0.2 to 0.8.

A second aspect of the invention relates to a lipid emulsion according to the first aspect and its embodiments for use in parenteral nutrition.

In certain embodiments, the parenteral nutrition is administered to a patient requiring short- or long-term Total Parenteral Nutrition (TPN). In certain embodiments, the parenteral nutrition is administered to a **TPN** patient with metabolic disease, specifically insulin resistance. In certain embodiments, the parenteral nutrition is administered to a **TPN** patient with a disease of the liver. In certain embodiments, the parenteral nutrition is administered to a **TPN** patient with systemic acute and/or chronic inflammation. In certain embodiments, the parenteral nutrition is administered to a **TPN** patient with compromised immune system and reduced host defense. In certain embodiments, the parenteral nutrition is administered to a patient sepsis patient. In certain embodiments, the parenteral nutrition is administered to a **TPN** patient undergoing chemotherapy.

An alternative of the second aspect of the invention relates to a lipid emulsion according to the first aspect and its embodiments for use in enteral nutrition.

In certain embodiments, the enteral nutrition is administered to a patient requiring short- or long-term enteral nutrition. In certain embodiments, the enteral nutrition is administered to a patient with metabolic disease, specifically insulin resistance. In certain embodiments, the enteral nutrition is administered to a patient with a disease of the liver. In certain embodiments, the enteral nutrition is administered to a patient with systemic acute and/or chronic inflammation. In certain embodiments, the enteral nutrition is administered to a patient with compromised immune system and reduced host defense. In certain embodiments, the enteral nutrition is administered to a patient sepsis patient. In certain embodiments, the enteral nutrition is administered to a patient undergoing chemotherapy.

In certain embodiments, the lipid emulsion further comprises a pharmaceutical drug. In certain embodiments, the pharmaceutical drug has a molecular weight of <1000g/mol, particularly of <500g/mol, and falls under the Lipinsky Rules of Five.

In certain embodiments, the drug is selected from
- a lipophilic drug, particularly wherein the lipophilic drug is selected from diazepam, propofol, etomidate, alprostadil, dexamethasone, flurbiprofen, vitamins A, **D, E, K,** paclitaxel, cyclosporine, clarithromycin, phenobarbital, physostigmine, cinnarizine, chlorambucil, and docetaxel;
- an RNA based drug using lipid emulsions as vehicles;
- an RNA vaccine or a DNA vaccine and, optionally, an adjuvant.

A third aspect of the invention relates to a lipid emulsion according to the first aspect and its embodiments for use as a counteragent/detoxifyer ("lipid sink") in a treatment of intoxication caused by a lipophilic pharmaceutical drug or a lipophilic toxic compound.

A fourth aspect of the invention relates to a lipid emulsion according to the first aspect and its embodiments for use in protection against ischemia-reperfusion injury of vital organs. In certain embodiments, the vital organ is selected from heart, brain, liver, kidneys, and lungs.

A fifth aspect of the invention relates to a lipid emulsion according to the first aspect for use in prevention or treatment of diabetes mellitus type II.

In certain embodiments, the lipid emulsion is formulated for parenteral administration.

In certain embodiments, the lipid emulsion is formulated for enteral or oral administration.

### Medical treatment, Dosage Forms and Salts

Similarly, within the scope of the present invention is a method or treating a condition associated with incapability of ingesting food in a patient in need thereof, comprising administering to the patient a lipid emulsion according to the above description.

Similarly, a dosage form for the treatment of a condition associated with incapability of ingesting food is provided, comprising a non-agonist ligand or antisense molecule according to any of the above aspects or embodiments of the invention.

As used therein, "a condition associated with incapability of ingesting food" can relate to any condition in which the patient is transiently or permanently disabled to receive nutrients by via naturalis, i.e. ingestion. Such conditions include being unconscious including coma, being incapable of swallowing, for example caused by neurological disorders, having a blocked oesophageal passage, for example as a result of trauma, tumour disease or other conditions in which the oesophageal passage is restricted or disabled.

Indications for total or partial parenteral nutrition encompass a wide range of clinical conditions such as critically ill patients (trauma, surgery, sepsis, shock), patients on home parenteral nutrition because of chronic intestinal failure, cachectic cancer patients, patients with inflammatory bowel disease (Crohn's disease, ulcerative colitis), patients with gastrointestinal obstruction, high-output enterocutaneous fistula, or short-bowel syndrome, (mostly) geriatric patients with acute or chronic debilitating diseases who cannot meet nutritional requirements, and patients with intractable nausea and vomiting (hyperemesis gravidarum). Moreover, malnutrition (calories and/or protein related) is a common health care issue with a high prevalence among hospitalized patients (20-50%) and is clearly linked to higher health care costs because of increased complications, longer hospital stays, and higher use of home health care services. In critically ill patients, supplementary parenteral nutrition to enteral nutrition aiming to satisfy the increased caloric needs under stress is also thought to decrease complication rates and associated health care costs.

The skilled person is aware that any specifically mentioned drug compound mentioned herein may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

Dosage forms may be for parenteral administration. Optionally, a pharmaceutically acceptable carrier and/or excipient may be present.

### Pharmaceutical Compositions and Administration

Another aspect of the invention relates to a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

In certain embodiments of the invention, the compound of the present invention is typically formulated into pharmaceutical dosage forms to provide an easily controllable dosage of the drug and to give the patient an elegant and easily handleable product.

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease. The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of a lipid emulsion as identified herein, for use in a method of manufacture of a medicament for the treatment or prevention of a condition associated with incapability of ingesting food.

Similarly, the invention encompasses methods of treatment of a patient having been diagnosed with a disease associated with incapability of ingesting food. This method entails administering to the patient an effective amount of the lipid emulsion as identified herein.

Wherever alternatives for single separable features such as, for example, a lipid concentration or a medical indication are laid out herein as "embodiments", it is to be understood that such alternatives may be combined freely to form discrete embodiments of the invention disclosed herein. Thus, any of the alternative embodiments for a lipid concentration may be combined with any of the alternative embodiments of a medical indication mentioned herein.

The invention is further illustrated by the following examples and figures, from which further embodiments and advantages can be drawn. These examples are meant to illustrate the invention but not to limit its scope.

### Description of the Figures

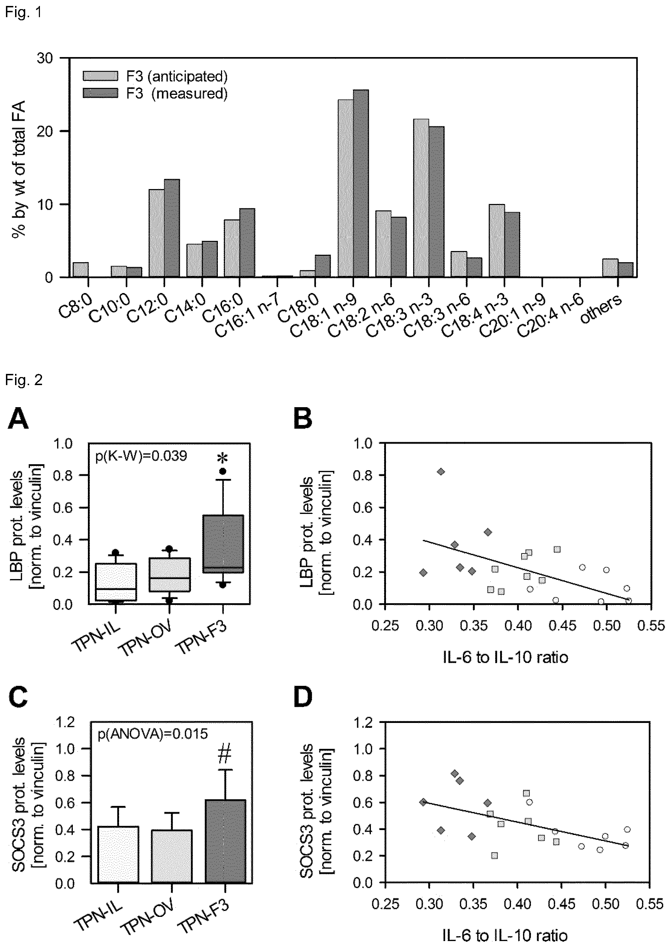

- Fig. 1: shows fatty acid composition of F3 (a novel lipid emulsion, TPN-F3, F3, W-TPN, and VV are used synonymously throughout this entire specification) as determined by gas chromatography.
- Fig. 2: shows Lipopolysaccharide-Binding Protein (LBP, panel A), correlation of LBP with IL-6 to IL-10 ratio (panel B), Suppressor Of Cytokine Signaling (SOCS3, panel C), and correlation of SOCS3 with IL-6 to IL-10 ratio (panel D) in total liver tissue of mice treated with different types of total parenteral nutrition (TPN) for 7 days. Lower IL-6 to IL-10 ratio indicates lower degree of inflammation in liver tissue. Protein abundance was determined by immunoblotting and normalized to vinculin. Y-axis indicates arbitrary relative units. TPN-IL=mice treated with Intralipid-based TPN. TPN-OV=mice treated with Omegaven-based TPN. TPN-F3=mice treated with F3-based TPN. *significantly increased to TPN-IL, #significantly increased to TPN-OV. Box plots show median and 25th and 75th percentile. Bars represent mean ± SD. N=7 for TPN-IL, N=7 for TPN-OV, N=6 for TPN-F3.

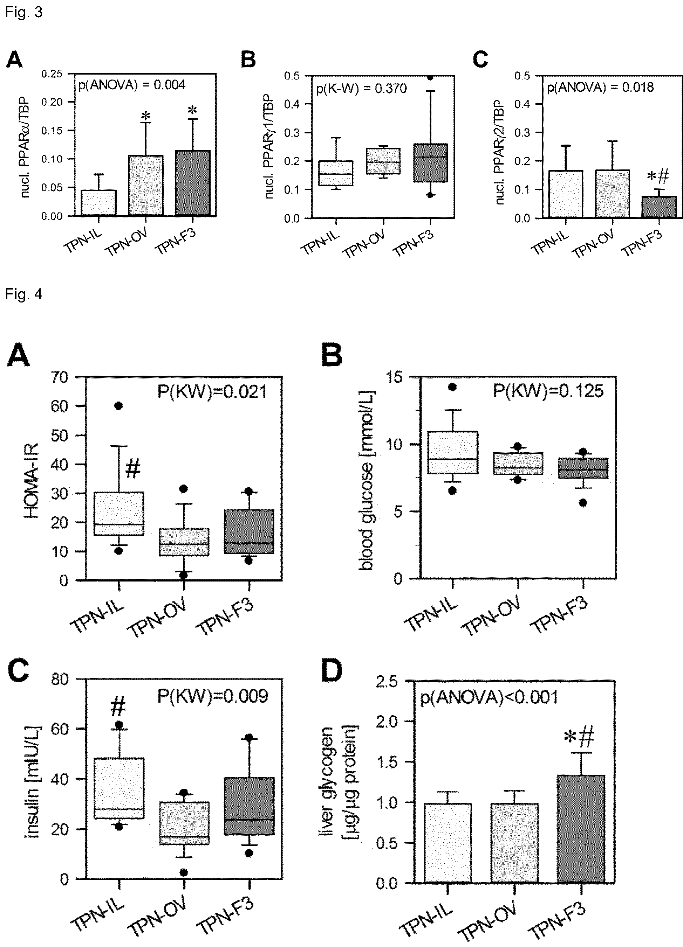

- Fig. 3: shows transcription factor PPARα (panel A), PPARγ1 (panel B), and PPARγ2 (panel C) in nuclear fractions from total liver tissue of mice treated with different types of total parenteral nutrition (TPN) for 7 days. Protein abundance was determined by immunoblotting and normalized to the nuclear marker TATA-binding protein (TBP). Y-axis indicates arbitrary relative units. TPN-IL=mice treated with Intralipid-based TPN. TPN-OV=mice treated with Omegaven-based TPN. TPN-F3=mice treated with F3-based TPN. *significantly different from TPN-IL, #significantly decreased to TPN-OV. Bars represent mean ± SD. Box plots show median plus 25th and 75th percentile. N=6 for each group.
- Fig. 4: shows HOMA-IR (homeostatic model assessment of insulin resistance, panel A), blood glucose (panel B), insulin plasma concentrations (panel C), and hepatic glycogen levels (panel D) in mice treated with different types of total parenteral nutrition (TPN) for 7 days. Outcomes were measured by standard methods (for details see reference#2). A higher HOMA-IR indicates reduced insulin sensitivity. HOMA-IR was calculated from plasma insulin and whole blood glucose using a normalization factor of 14.1, which is the adjusted factor for C57BL/6J mice. Liver glycogen is a reliable index of insulin signaling and insulin sensitivity in the liver: as higher the glycogen levels are, as more efficacious the insulin signaling is. TPN-IL=mice treated with Intralipid-based TPN. TPN-OV=mice treated with Omegaven-based TPN. TPN-F3=mice treated with F3-based TPN. #significantly increased to TPN-OV, *significantly increased to TPN-IL. Bars represent mean ± SD. Box plots represent median plus 25th and 75th percentile. N=12 for each group.

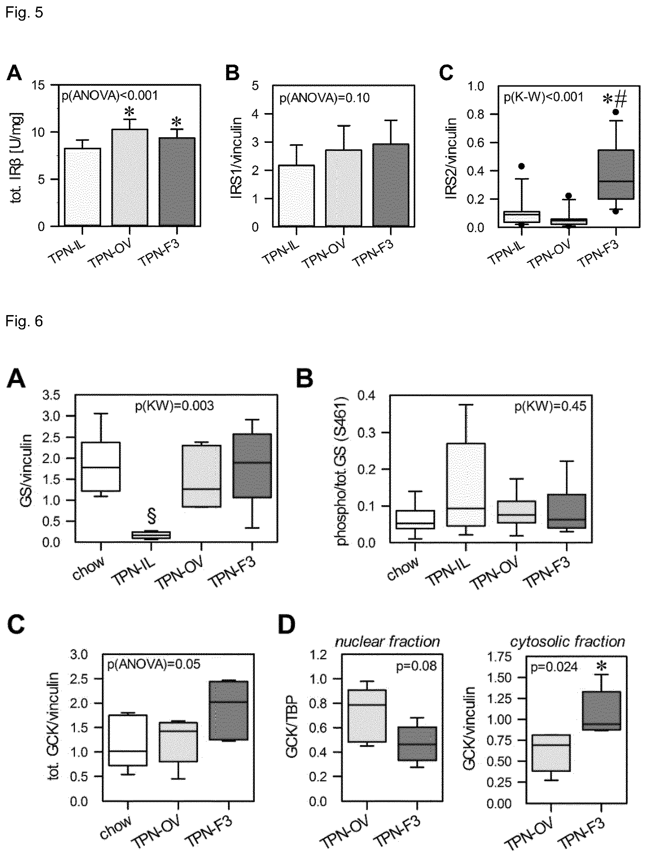

- Fig. 5: shows insulin receptor β subunit (IRβ, panel A), insulin receptor substrate 1 (IRS1, panel B), and insulin receptor substrate 2 (IRS2, panel C) abundance in total liver tissue homogenates of mice treated with different types of total parenteral nutrition (TPN) for 7 days. IRβ abundance was measured by enzyme-linked immunosorbent assay. IRS protein abundance was determined by immunoblotting and normalized to vinculin. Y-axis indicates arbitrary relative units. TPN-IL=mice treated with Intralipid-based TPN. TPN-OV=mice treated with Omegaven-based TPN. TPN-F3=mice treated with F3-based TPN. *significantly increased to TPN-IL, #significantly increased to TPN-OV. Bars represent mean ± SD. Box plots represent median plus 25th and 75th percentile. N=12 for each group.
- Fig. 6: shows glycogen synthase (GS, panel A), glycogen synthase phosphorylation at serine 641 (panel B), glucokinase (GCK, panel C) and its nuclear (inactive) and cytosolic (active) fractions (panel D) in liver tissue of mice treated with different types of total parenteral nutrition (TPN) for 7 days. Protein abundance was determined by immunoblotting and normalized to vinculin or TBP for nuclear fractions, respectively. Chow=tissue samples from chow-fed mice served as control samples. TPN-IL=mice treated with Intralipid-based TPN. TPN-OV=mice treated with Omegaven-based TPN. TPN-F3=mice treated with F3-based TPN. §significantly reduced vs all other groups. *significantly increased vs TPN-OV. Box plots represent median plus 25th and 75th percentile. N=6 for each group.

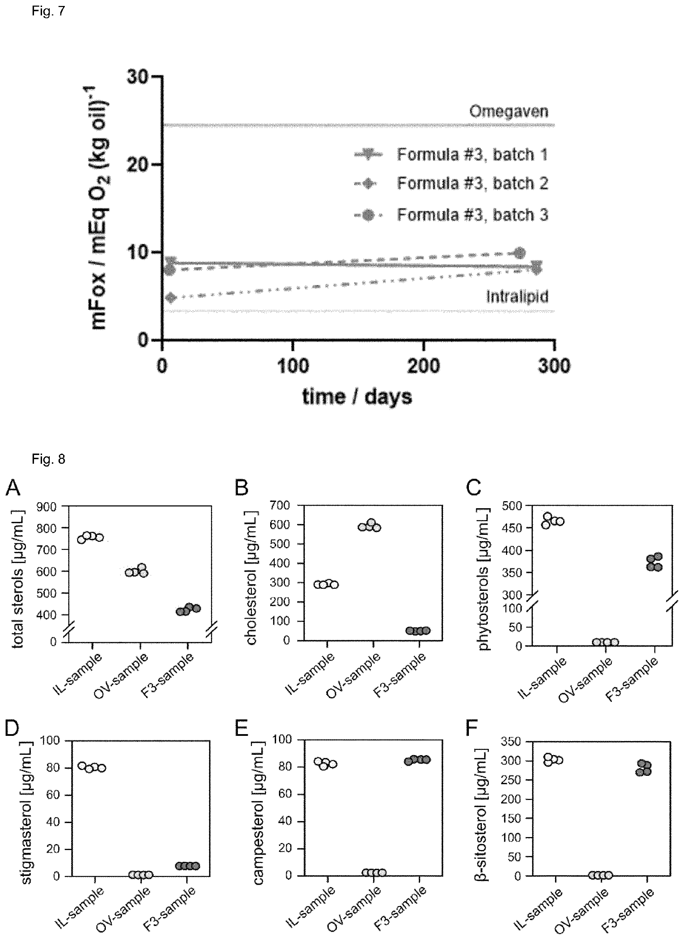

- Fig. 7: shows Ferrous Oxidation-Xylenol Orange (FOX) Assay results. Content of primary oxidation products over time in Intralipid, Omegaven, and 3 different batches of F3 lipid emulsion.
- Fig. 8: shows sterol content in Intralipid (IL), Omegaven (OV), and Formula #3 (F3) (panel A). Plant sterols and plant stanols (phytosterols, panel C) are plant-derived compounds that are structurally related to cholesterol (panel B), a sterol of animal origin). The higher levels of cholesterol in Intralipid (as compared to F3), may be due to the purification process used for the egg lecithin in Intralipid (as compared to Lipoid 80 used in F3). Stigmasterol (panel D) is an unsaturated phytosterol typically occurring in soybeans. Other phytosterols include campesterol (panel E) and β-sitosterol (panel F).

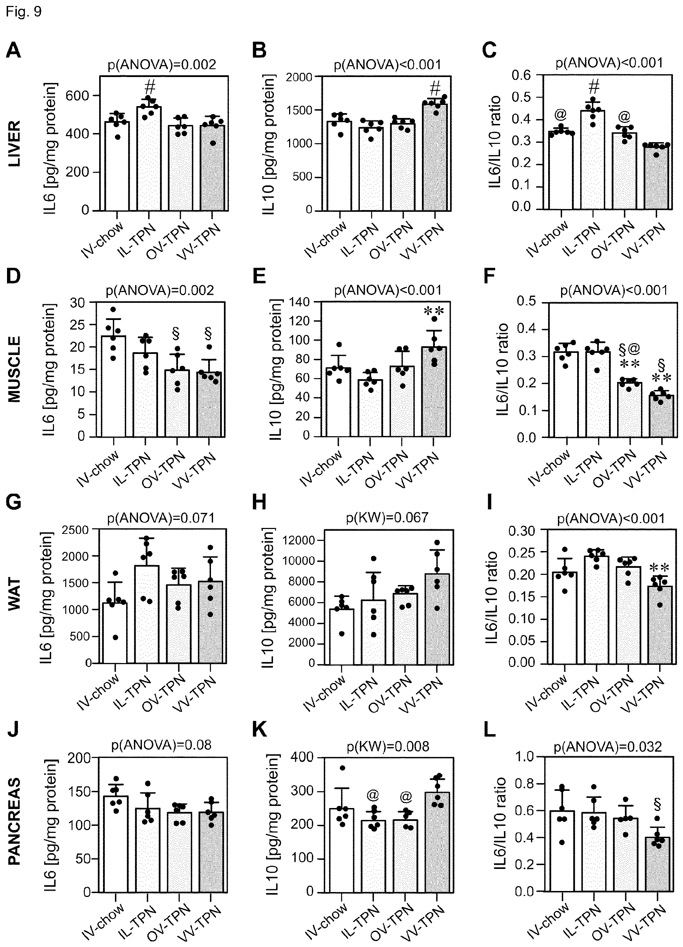

- Fig. 9: shows anti-inflammatory actions of TPN-F3 in key insulin-sensitive tissues. Interleukin-6 (IL6; panel A, D, G, J), interleukin-10 (IL10; panel B, E, H, K), and IL6/IL10 ratio (panel C, F, I, L) in total tissue homogenates of liver (panel A, B, C), skeletal muscle (panel D, E, F), epididymal white adipose tissue (WAT) (panel G, H, I), and pancreas (panel J, K, L) from mice treated with different types of total parenteral nutrition (TPN) for 7 days. Cytokine concentrations were measured by enzyme-linked immunosorbent assays. IV-chow, mice on chow diet and continuous saline infusion; IL-TPN, mice treated with Intralipid-based TPN; OV-TPN, mice treated with Omegaven-based TPN; W-TPN, mice treated with TPN-F3-based TPN. #, significantly increased vs all other groups; @, significantly different from W-TPN, **, significantly different from IL-TPN; §, significantly reduced vs. IV-chow. Bars represent mean±SD. N=6 per group.

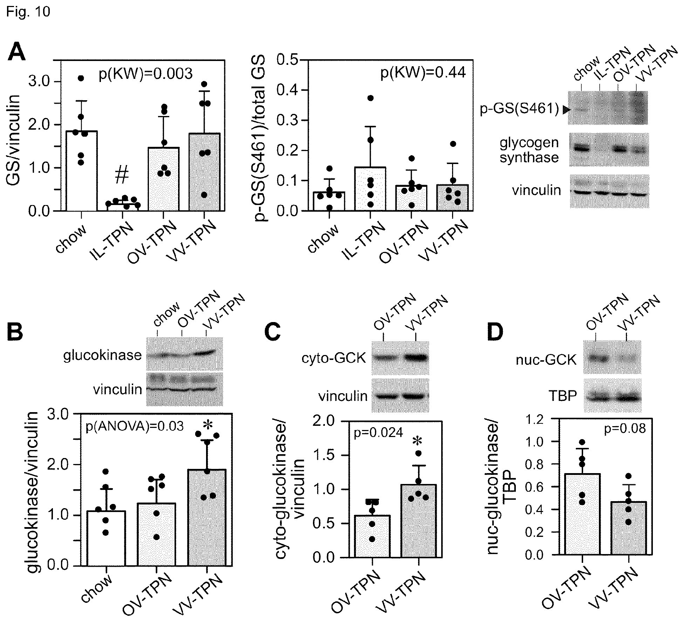

- Fig. 10: shows regulation of glycogenesis in different TPN regimens. Panel A: Glycogen synthase (GS), GS phosphorylation at serine 641 and representative immunoblots in liver tissue of mice treated with different types of total parenteral nutrition (TPN) for 7 days. Panel B-D: Abundance of glucokinase (GCK) in total tissue lysates (panel B), in the cytosolic (active) fraction (panel C), and in the nuclear (inactive) fraction (panel D) with representative immunoblots. Protein expression was normalized to vinculin for total tissue lysates or cytosolic fractions or TATA-binding protein (TBP) for nuclear fractions, respectively. Chow, chow-fed mice (control samples); IL-TPN, mice treated with Intralipid-based TPN; OV-TPN, mice treated with Omegaven-based TPN; VV-TPN, mice treated with TPN-F3-based TPN. #significantly reduced vs all other groups; *significantly increased vs chow; **significantly increased vs TPN-OV. Bars represent mean±SD. N=6 per group.

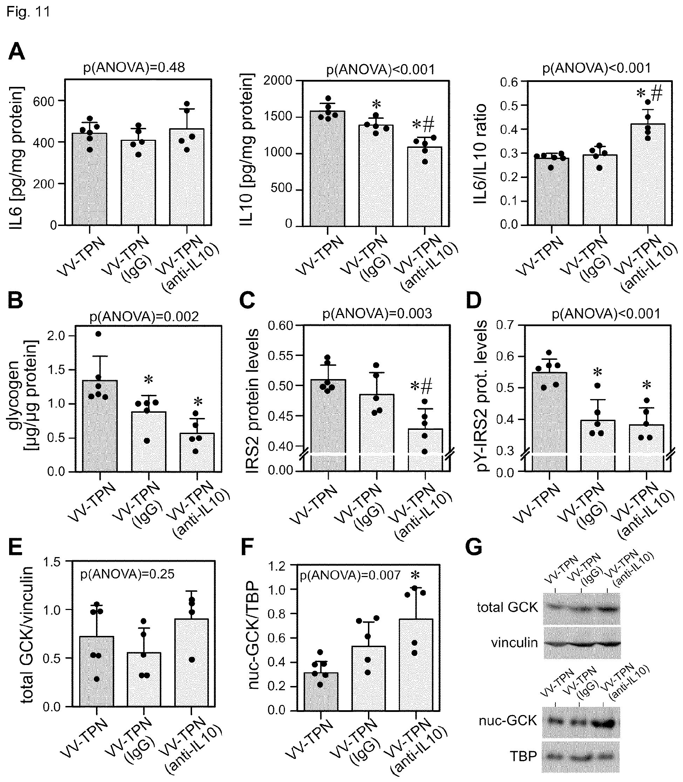

- Fig. 11: shows interleukin-10 mediated insulin signaling in TPN-F3-based TPN. Effects of the neutralizing anti-IL10 treatment on IL6, IL10, and respective ratio (panel A), liver glycogen levels (panel B), IRS2 protein expression and its tyrosine phosphorylation (panel C and D), glucokinase (GCK) in total tissue lysates (panel E and G), as well as inactive (nuclear) GCK (panel F) in mice treated with W-TPN for 7 days. Protein abundance was determined by immunoblotting and normalized to vinculin and TATA-binding protein (TBP) for nuclear fractions, respectively. W-TPN, mice treated with TPN-F3-based TPN; W-TPN (IgG), mice treated with W-based TPN and concomitant isotype control antibody; W-TPN (anti-IL10), mice treated with W-based TPN and concomitant neutralizing anti-IL10. *significantly different from W-TPN. #significantly different from W-TPN (IgG). Bars represent mean±SD. N=5-6 for each group. Note: isotype control antibody IgG has been previously shown to exert some anti-IL10 effects through Fc receptor binding to immune cells.

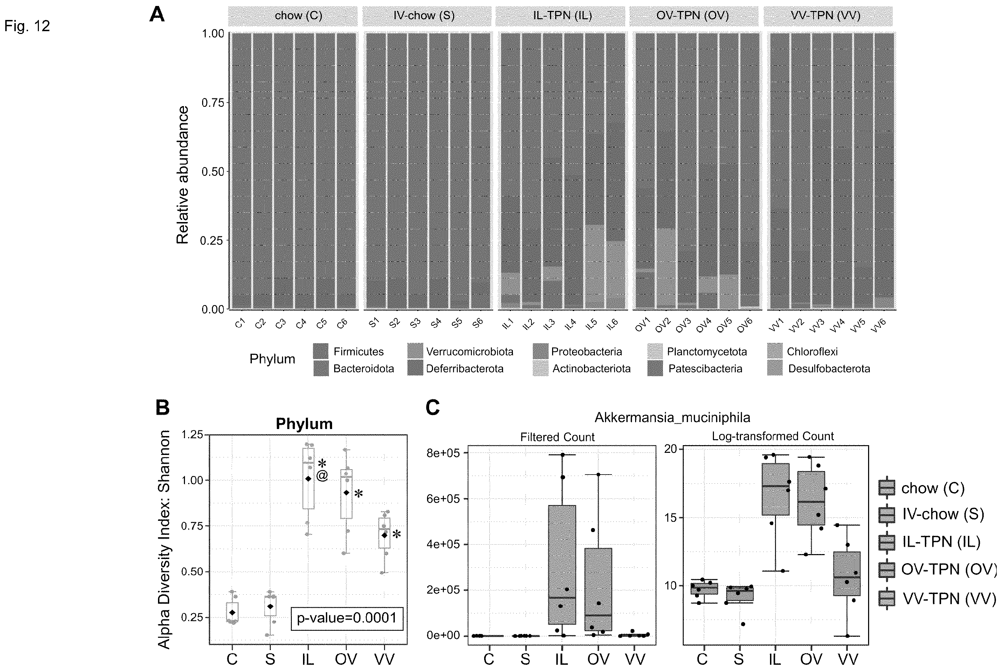

- Fig. 12: shows microbiome analysis of bowel mucosal samples from the colon of mice treated with IL-TPN, OV-TPN, and VV-TPN for 7 days as compared to control mice. Panel A: Relative bacterial abundance at the *Phylum* level is altered by TPN with a significant expansion of the *Phylum Bacteroidota* at the cost of *Firmicutes.* Panel B: Alpha diversity at the *Phylum* level was significantly affected by TPN containing Intralipid (IL-TPN) and Omegaven (OV-TPN), but to a significantly lesser extent by TPN-F3 (W-TPN). Panel C: *Verrucomicrobiota,* namely *Akkermansia muciniphila* significantly increased in mice receiving IL-TPN and OV-TPN as compared to mice receiving W-TPN. chow (C), control mice on chow diet; IV-chow (S), mice on chow diet and continuous saline infusion; IL-TPN (IL), mice treated with Intralipid-based TPN; OV-TPN (OV), mice treated with Omegaven-based TPN; W-TPN (VV), mice treated with TPN-F3-based TPN. *, significantly different from chow and IV-chow; @, significantly different from VV-TPN, N=6 per group. Figures were generated using MicrobiomeAnalyst.

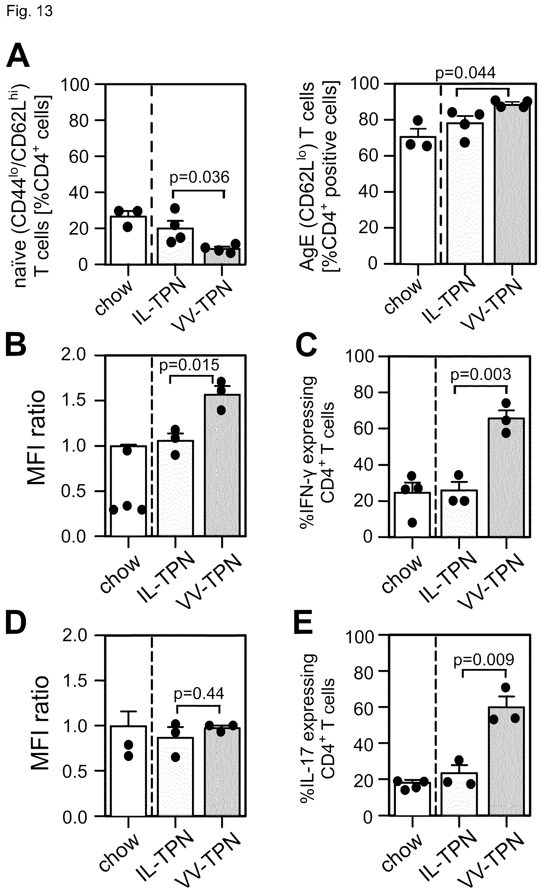

- Fig. 13: shows CD4⁺ T-cells characteristics in liver tissue of W-TPN mice. Panel A: Proportions of naïve (cD44^{low}/CD62L^{high}) and of "antigen-experienced" T-cells, i.e. cells expressing low amounts of CD62L/L-selectin including effector memory (CD44^{high}/CD62L^{low}) T-cells. Note that the abundance of central memory (CD44^{high}/CD62L^{high}) CD4⁺ T-cells is very low in the liver. Panel B-D: Intracellular staining for production of cytokines in hepatic CD4⁺ T-cells in response to activation by phorbol myristate acetate (PMA) and ionomycin. Intracellular staining was performed to measure expression of interferon-y (IFNγ; panel B and C) and of interleukin-17A (IL17A; panel D and E) produced by CD4⁺ T cells. Data are presented as mean fluorescence intensity ratio (MFI ratio; panel B and D) and percentage positive-stained CD4⁺ T cells (% CD4⁺ T-cells; panel C and E), respectively. chow, control mice on chow diet; IL-TPN, mice treated with Intralipid-based TPN; W-TPN, mice treated with TPN-F3-based TPN. Bars represent mean±SE. Dots represent individual experiments. N=3-4 for each group.

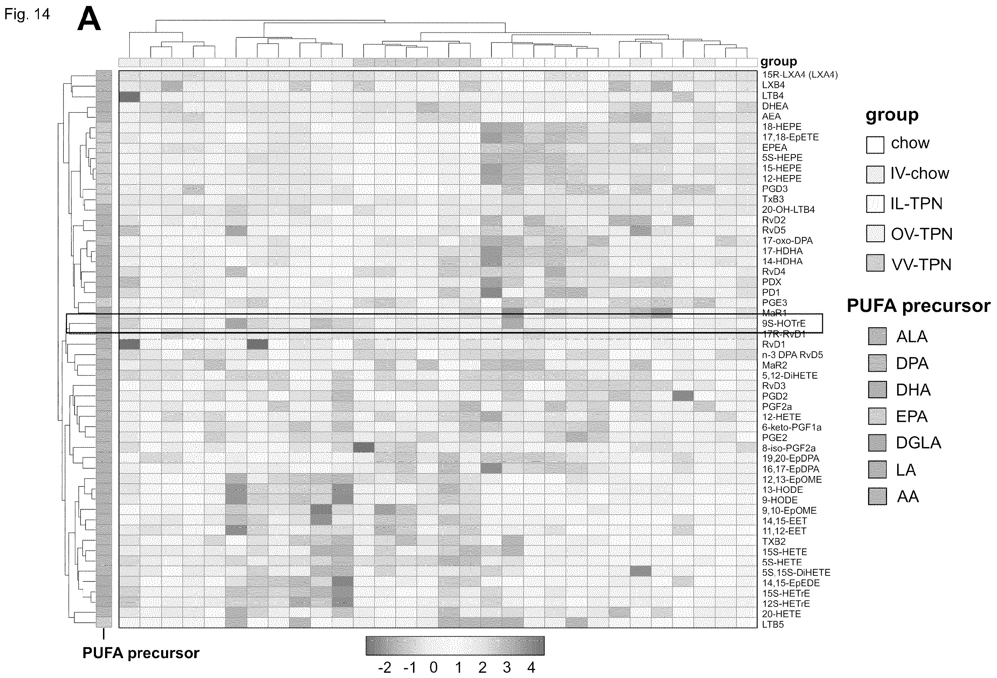

- Fig. 14: shows lipid mediators in liver tissue of mice treated with different TPNs. Panel A: Heatmap of the measured lipid mediators. Columns correspond to study groups, and rows to lipid mediators derived from the polyunsaturated fatty acids (PUFAs) precursors annotated on the left side of the heatmap. Data are color-coded according to z-scores representing the relative amount of each lipid mediator. n-3 PUFA precursors are α-linolenic acid (ALA), docosapentaenoic acid (DPA), eicosapentaenoic acid (EPA), and docosahexaenoic acid (DHA). n-6 PUFA precursors are dihomo-γ-linolenic acid (DGLA), linoleic acid (LA), and arachidonic acid (AA). 9S-hydroxy-9Z,11E,15Z-octadecatrienoic acid (9(S)-HOTrE), a 15-lipoxygenase metabolite of ALA, is highlighted.

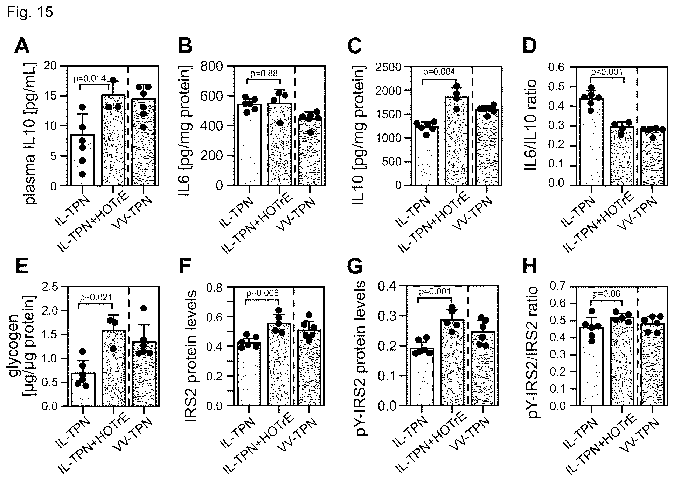

- Fig. 15: shows supplementation of 9(S)/13S-hydroxy-9Z,11E,15Z-octadecatrienoic acid (9(S)/13(S)-HOTrEs) to Intralipid-based TPN mimics the phenotype elicited by TPN-F3-based TPN. Mice receiving IL-TPN were treated with 9/13-hydroxy-octadecatrienoic acids (5 ng/mL) added to the TPN mixture. Plasma concentrations of interleukin-10 (IL10; panel A), tissue levels of interleukin-6 (IL6; panel B), interleukin-10 (panel C), IL6/IL10 ratio (panel D) in total liver tissue homogenates. Liver glycogen content (panel E), abundance of Insulin Receptor Substrate 2 protein (IRS2; panel F), of tyrosine-phosphorylated IRS2 (panel G), and respective ratio (panel H), IL-TPN, mice treated with Intralipid-based TPN; W-TPN, mice treated with TPN-F3-based TPN; IL-TPN+HOTrE, mice treated with Intralipid-based TPN supplemented with 9(S)/13(S)-HOTrE. Bars represent mean±SD. N=4-6 per group.

- Table 1: shows composition of the oily phase in commonly used lipid emulsions for total parenteral nutrition (quantities given per 100 mL). Emulsions are 20% with the exception of Omegaven, which is only available as 10% emulsion. Quantities are given per 100 mL. Intralipid, SMOFlipid, and Omegaven are manufactured by Fresenius Kabi (Bad Homburg, Germany); Lipofundin by B. Braun (Melsungen, Germany); ClinOleic by Baxter Healthcare Corporation (Deerfield, IL, USA). All emulsions use egg yolk lecithin as emulsifier and glycerol to adjust for osmolarity. SFA, saturated fatty acids; MUFA, monounsaturated fatty acids; PUFA, polyunsaturated fatty acids.
- Table 2: shows composition of F3 listing the major fatty acids present in the lipid emulsion

### Examples

In the following, TPN-F3, F3, W-TPN, and VV are used synonymously.

### Example 1: Composition of the oily phase in commonly used lipid emulsions

**Table 1: Composition of the oily phase in commonly used lipid emulsions for total parenteral nutrition (quantities given per 100 mL)**

| | **Intralipid 20%** | **Omegaven 10%** | **SMOFlipid 20%** | **Clinoleic 20%** | **Lipofundin 20%** |
|---|---|---|---|---|---|
| Soybean oil | 20 g (100%) | 0 g | 6 g (30%) | 4 g (20%) | 10 g (50%) |
| Olive oil | 0 g | 0 g | 5 g (25%) | 16 g (80%) | 0 g |
| Coconut oil | 0 g | 0 g | 6 g (30%) | 0 g | 10 g (50%) |
| Fish oil | 0g | 10 g (100%) | 3 g (15%) | 0 g | 0 g |
| PUFA g% | 60% | 58% | 29% | 20% | 30% |
| MUFA g% | 24% | 26% | 30% | 63% | 12% |
| SFA g% | 16% | 16% | 41% | 17% | 58% |
| n-6 fatty acids g% | 53% | ~6-7% | ~20% | 19% | 26% |
| n-3 fatty acids g% | 7.8% | 52% | ~8% | 2% | 4% |
| n-6 to n-3 ratio | 7:1 | 1:8 | ~2.5:1 | 9:1 | ~7:1 |

SMOFlipid was created to account for recommendations regarding the optimal dietary intake of polyunsaturated fatty acids whereby the n-6:n-3 ratio should be between 1:1 and 4:1. The oily phase of the newly developed F3 also accounts for these dietary requirements and displays a n-6:n-3 ratio of 1:2.6. However, it consists of non-GMO (without genetic engineering) vegetable oils only, one of which is Ahiflower oil. Ahiflower oil is a rich single-plant source of n-3 alpha-linolenic acid (ALA) and stearidonic acid (SDA) as well as n-6 gamma linolenic acid (GLA) and linoleic acid (LA). Ahiflower oil contains 17-20% SDA, the highest level of naturally occurring SDA of any commercially available dietary plant oil. Oils with high SDA content are increasingly recognized as an excellent source of n-3 fatty acids affecting inflammation and metabolism. Moreover, SDA is less unsaturated than eicosapentaenoic acid (EPA) and docosahexaenoic acid (DHA) present in fish oil, making it more stable when present in lipid emulsions. The oil blend of F3 contains 50% Ahiflower and, consequently, approximately 10% SDA and approximately 20% ALA. See Details in the **Table 2** below.

**Table 2: Composition of F3 listing the major fatty acids present in the lipid emulsion**

| | **F3 (20%)** | **Fatty acid (notation)** | **approx. % amount** |
|---|---|---|---|
| Olive oil | 5 g (25%) | Caproic, caprylic, and capric (C6:0, C8:0, C10:0) | ~3.5% |
| Coconut oil | 5 g (25%) | Lauric (C12:0) | 12% |
| Ahiflower oil | 10 g (50%) | Myristic (C14:0) | 4.5% |
| PUFA g% | 44% | Palmitic (C16:0) | 7.9% |
| MUFA g% | 27% | Oleic (C18:1) | ~24% |
| SFA g% | 29% | Linoleic (C18:2 n-6) | 9% |
| n-6 fatty acids g% | 12% | α-Linolenic (C18:3 n-3) | ~22% |
| n-3 fatty acids g% | 32% | Stearidonic (C18:4 n-3) | 10% |
| n-6 to n-3 ratio | 1:2.6 | γ-Linolenic (C18:3 n-6) | 3% |

F3 also contains EDTA (2.5 µM) and tocopherols (160-200 mg/L) to stabilize the unsaturated fatty acids and to protect against oxidation.

### Example 2: Problems generally associated with TPN

The infusion of lipid emulsions with provision of fatty acids generally causes insulin resistance, promotes accumulation of lipids with enhancement of tissue inflammation (specifically interleukin-6 release). Moreover, the use of lipid emulsions in TPN is associated with impaired immune responses and a higher incidence of infections. For a detailed review see Lucchinetti et al. Novel Strategies to Prevent Total Parenteral Nutrition-Induced Gut and Liver Inflammation, and Adverse Metabolic Outcomes. Mol Nutr Food Res. 2021;65(5): e1901270. doi: 10.1002/mnfr.201901270. PMID: 32359213. F3 is a newly developed lipid emulsion with unique beneficial effects on inflammation, insulin signaling, and immune responses and reduced adverse effects specifically when used in the context of TPN.

### Example 3: TPN-F3 elicits strong anti-inflammatory actions in key metabolic insulin-sensitive tissues during TPN compared with standard lipid emulsions

After seven days of TPN, body weight was similar in TPN groups and consistent with previous reports using the same TPN mouse model. W-TPN increased the plasma concentration of IL10 compared with IL-TPN and OV-TPN. W-TPN further reduced the concentration of the proinflammatory cytokine IL6 in liver tissue and simultaneously increased the production of the anti-inflammatory cytokine IL10, markedly reducing the IL6/IL10 ratio **(****Figure 9****).** IL6/IL10 ratios were higher in liver tissues of IL-TPN and OV-TPN mice. A similar pattern of the IL6/IL10 ratio was observed in skeletal muscle, epididymal white adipose (eWAT) and pancreatic tissues **(****Figure 9****).** W-TPN also reduced TNFα in liver tissue when compared with IL-TPN. W-TPN, but not IL-TPN or OV-TPN, increased the production of Lipopolysaccharide-Binding Protein (LBP) in the liver, a protein which detoxifies endotoxin (LPS) via high density-lipoprotein (HDL) and chylomicrons. Higher LBP levels correlated with lower inflammation, as evidenced by lower IL6/IL10 ratios, implying that LBP scavenged endotoxin released from the leaky gut resulting in reduced production of IL6 in the liver **(****Figure 2A** **and** **B****).** Likewise, Suppressor Of Cytokine Signaling (SOCS3), a protein which inhibits IL6 signaling, was increased in livers of W-TPN mice and inversely correlated with IL6/IL10 ratios **(****Figure 2C** **and** **D****).** W-TPN, similar to OV-TPN, elevated hepatic nuclear PPARα, a transcription factor known to increase hepatic fatty acid oxidation and to reduce fat-induced liver inflammation, while no differences in nuclear PPARγ1 were observed among the different TPN groups **(****Figure 3A** **and** **B****).** Only W-TPN reduced nuclear PPARγ2, a transcription factor known to foster lipid accumulation and proinflammatory cytokine production in the liver and to cause steatohepatitis **(****Figure 3C****).** W-TPN and OV-TPN also reduced LPS content in epididymal white adipose tissue (eWAT) when compared with IL-TPN, but only W-TPN markedly reduced the protein expression of the pro-inflammatory NFₖB in eWAT, consistent with its decreased IL6/IL10 ratio. Finally, W-TPN also increased anti-inflammatory IL4 in liver and in pancreatic tissue. In summary, W-TPN when compared with IL-TPN and OV-TPN exerted the strongest anti-inflammatory actions in multiple key metabolic insulin-sensitive tissues.

### Example 4: F3 fosters insulin signaling, specifically in the liver, and further improves glucose tolerance in the whole body during TPN

TPN impairs insulin signaling resulting in whole body insulin resistance. TPN-F3 and TPN-OV improve whole-body insulin response as assessed my HOMA-IR (homeostatic model assessment for insulin resistance) when compared to TPN-IL **(****Figure 4A-C).** However, only TPN-F3 preserves insulin signaling in the liver as evidenced by normalized levels of glycogen, a reliable index of hepatic insulin sensitivity **(****Figure 4D****).** TPN-F3 and TPN-OV increase the abundance of the insulin receptor in liver tissue when compared with TPN-IL **(****Figure 5A****),** but only TPN-F3 increases the abundance of the insulin receptor substrate 2 (IRS2), while IRS1 remains unchanged **(****Figure 5B and 5C** **and Table 4).** TPN-IL markedly reduces the abundance of the glycogen synthase in liver tissue, while TPN-F3 and TPN-OV do not **(****Figure 10A****).** No differences in the phosphorylation state of hepatic glycogen synthase at Ser461, a phosphorylation site which causes its inhibition, are noted between the three TPNs **(****Figure 10A****).** However, TPN-F3 shows higher abundance of glucokinase **(****Figure 10B****),** the rate-limiting step of glycogen formation in the liver, and the fraction of active cytosolic glucokinase is higher in TPN-F3 when compared with TPN-OV **(****Figure 10C****),** explaining the higher glycogen storage in liver tissue of mice treated with F3. Together, these findings highlight the enhanced insulin sensitivity in the liver with TPN-F3 when compared with TPN-IL or TPN-OV. Administration of a neutralizing IL-10 antibody to TPN-F3 increases the IL-6/IL-10 ratio to values observed in TPN-IL **(****Figure 11A****)** and diminishes hepatic glycogen storage **(****Figure 11B****)** because of increased nuclear fraction of glucokinase **(****Figure 11F****),** consistent with impaired insulin signaling. Collectively, these findings emphasize the key role of the elevated IL-10 production in liver tissue for preserving insulin signaling in mice treated with TPN-F3, and further mechanistically link the metabolic benefits from TPN-F3 to its anti-inflammatory cytokine profile.

**Table 4: Metabolic data**

| | chow | IV-chow | IL-TPN | OV-TPN | W-TPN | p-value |
|---|---|---|---|---|---|---|
| Blood glucose, fed [mmol/L] | 10.4 (9.7, 12.1) | 9.9 (9.1, 10.1) | 9.7 (8.1, 12.1) | 8.7 (7.7, 9.7) | 8.2 (7.6, 8.8) | 0.25 |
| Insulin [µIU/L] | 7.9 (7.0, 12.3) | 9.7 (7.9, 13.5) | 49.1 (25.2, 59.4) + | 18.5 (13.8, 26.6) | 28.7 (16.5, 50.5) | **0.036** |
| Glucagon [pmol/L] | 3.2 (1.3) | 3.0 (0.7) | 3.3 (0.9) | 2.4 (0.8) | 4.4 (1.5)+ | **0.026** |
| GLP-1 [pmol/L] | 51.2 (44.3, 64.3) | 50.6 (42.5, 63.6) | 63.7 (52.0, 72.6)+ | 43.1 (31.0, 55.1) | 63.2 (60.6, 64.0) | **0.024** |
| murine HOMA-IR | | | 29.9 (19.3, 32.7)+ | 11.5 (8.8, 15.6) | 15.8 (9.9, 28.8) | **0.018** |
| Liver glycogen | 1.21 (0.17) | 1.35 (0.32) | 0.70 (0.25) | 0.96 (0.17) | 1.28 (0.25) § | **0.001** |
| [µg/µg protein] | | | | | | |
| Liver total IRβ [U/mg] | 9.3 (1.5) | 8.3 (1.1) | 8.3 (0.9)* | 10.1 (0.9) | 9.4 (0.9) | **<0.001** |
| IRS2 protein levels [OD] | 0.62 (0.05) | 0.65 (0.06) | 0.67 (0.07) | 0.67 (0.06) | 0.90 (0.16)# | **<0.001** |
| pY-IRS2/IRS2 | 0.33 (0.03) | 0.31 (0.04) | 0.34 (0.03) | 0.32 (0.04) | 0.35 (0.03) | 0.36 |

### Example 5: F3, but not IL or OV, promotes strong host defense against invasive bacteria such as Akkermansia muciniphila during TPN

TPN causes depletion of immune cells and dysfunctional cytokine responses. However, F3-TPN as opposed to TPN-IL induces an "activated" phenotype of resident liver macrophages (Kupffer cells) with M₁-like polarization and increases the percentage of INFγ and IL17 producing CD4⁺ T-cells **(****Figure 13****),** consistent with previously reported higher immune responses in insulin-sensitive as opposed to insulin-resistant macrophages and CD4⁺ T-cells. In accordance with this observation, hepatic tissue concentrations of INFγ, a key cytokine for efficacious host defense, are also elevated in mice treated with TPN-F3 when compared with mice treated with TPN-IL or TPN-OV.

A detailed gut microbiome analysis based on 16S RNA sequencing was performed. To this purpose, mucosal samples from the colon were collected at the end of the 7-day experimental period. DNA was extracted following the protocol of ZymoBIOMICS DNA Miniprep Kit (D4300, Zymo Research Corp; Irvine, CA, USA). The amplification of the V3-V4 hypervariable region was performed with standard primers 341F and 805R. Libraries were prepared using the Metafast protocol, at Fasteris facilities (Genesupport/Fasteris SA, Plan-les-Ouates, Switzerland). 16S rRNA gene sequences were clustered into Operational Taxonomic Units (OTUs) and mapped to the SILVA Database. All data analysis was performed using the web-based tool MicrobiomeAnalyst.ca (https://www.microbiomeanalyst.ca/). The following results were obtained. As previously shown, TPN reduces the abundance of Firmicutes and increases the abundance of Bacteroidota **(****Figure 12****).** This analysis further reveals significant changes in the phylum *Verrucomicrobiota.* The phylum encompasses and is defined by the genus *Akkermansia,* of which the mucin-degrading bacterium *Akkermansia muciniphila* is the sole and most prominent member. *Akkermansia muciniphila* is a commensal, which becomes pathogenic during gut dysbiosis and IL-10 deficiency. Of importance, it is inhibited from invading the gut wall in mice treated with TPN-F3 as opposed to mice treated with TPN-IL or TPN-OV, providing evidence for a stronger host defense when treated with TPN-F3 **(****Figure 12****).**

To better understand the effects of TPNs with different lipid emulsions on host defense, immune cell abundance and phenotypes were determined in tissues. IL-TPN was chosen for direct comparison with W-TPN because of its clearly distinct immune-metabolic phenotype as opposed to OV-TPN, which showed an intermediate phenotype. TPN generally caused a decrease in leukocytes (CD45+) in various organs including the bowel, which typically undergoes marked atrophy. Immune cell profiling of spleen, mesenteric lymph nodes, and liver showed defective TNFα-response in PMA/ionomycin-stimulated CD4⁺ T-cells (except for W-TPN in the liver) when compared with chow-fed mice. However, W-TPN as opposed to IL-TPN increased the percentage of antigen experienced, i.e., primed effector and effector memory CD4⁺ T-cells (CD44^{low}/CD62L^{low}, CD44^{high}/CD62L^{low}) **(****Figure 13A****)** and IFNγ- **(****Figure 13B and 13C****)** and IL17-producing CD4⁺ T-cells **(****Figure 13D and 13E****),** activated B-cells (CD80+/CD86+), and concomitantly increased the percentage of M1-like resident (CD11b^{low}/CD11c+) and nonresident (CD11b^{high}/CD11c+) macrophages in the liver, while reducing M2-like macrophages (CD11b^{low}/CD206+, CD11b^{high}/CD206+). Activation of B-cells was associated with higher levels of IgG against endotoxin (LPS) in W-TPN when compared with OV-TPN and IL-TPN. In accordance with cellular findings, hepatic tissue concentrations of IFNγ, a key cytokine for efficacious host defense, were higher in mice treated with W-TPN when compared with IL-TPN and OV-TPN. Although we could not reliably measure intracellular IL10 expression in immune cells using flow cytometry, possibly because of lower intracellular expression, T-cells and macrophages collected after seven days of W-TPN both showed expression of IL10 as measured by immunoblotting. IL6, IFNγ and TNFα expression in macrophages, as measured by flow cytometry, were similar in chow-fed, IL-TPN and W-TPN mice.

### Example 6: TPN-F3 elicits a distinct profile of lipid mediators in liver tissue and 9-hydroxy-octadecatrienoic acid, a lipid mediator of 18-carbon ALA, contributes to the TPN-F3-induced immuno-metabolic phenotype

Oxylipins are oxygenated metabolites of fatty acids generated in the liver including eicosanoids (prostaglandins and leukotrienes) and specialized pro-resolving mediators that are known as potent regulators of the metabolism and immune responses. To test whether the provision of a higher amount of 18-carbon *n-3* fatty acids, namely ALA and SDA released during W-TPN, elicited a distinct profile of oxylipins in the liver, lipid mediators were determined using a targeted lipidomics approach (UHPLC-MS/MS). While OV-TPN increased many oxylipins derived from long-chain *n-3* fatty acids, namely EPA and DHA, with known (mainly) ant-inflammatory actions, W-TPN did not, but also showed no increases in many common pro-inflammatory oxylipins derived from arachidonic acid, as observed in IL-TPN. 9S-hydroxy-10E,12Z,15Z-octadecatrienoic acid (9-HOTrE), a monohydroxy polyunsaturated fatty acid generated from ALA by 12/15-lipoxygenases, was exclusively increased in W-TPN mice **(****Figure 14****).** To test whether hydroxy-octadecatrienoic acids contributed to the immune-metabolic phenotype induced by W-TPN, 9/13-HOTrEs were added to IL-TPN. Provision of these lipid mediators increased IL10 plasma and liver tissue concentrations after TPN, resulting in similarly low IL6/IL10 ratio as observed in liver tissue of VV-TPN mice **(****Figure 15D****).** The reduced IL6/IL10 ratio was further accompanied by increased IRS2 protein expression and its tyrosine phosphorylation and higher glycogen contents in the livers **(****Figure 15F****).** Supplementation of HOTrEs also increased IFNγ concentrations in liver tissue when added to IL-TPN, while TNFα concentrations remained elevated as with IL-TPN. These observations provide evidence that HOTrEs indeed contribute to the immune-metabolic phenotype induced by W-TPN.

### Example 7: Additional benefits of F3 when compared with commercially available lipid emulsions

### Sustainability

F3 does not contain fish oil, which is of concern because of overfishing oceans. Also, there are no accumulated toxins (such as radioactive substances from the Fukoshima nuclear disaster in 2011) from the food chain.

### Resistance to oxidation/degradation

F3 has fatty acids with only 4 double bonds as opposed to 5 or 6 double bonds present in fish oil and thus is more resistant to oxidation/degradation (prolonged shelf-life). Notably, F3 shows 2.5x lower primary oxidation products than Omegaven over an extended period of time (see **Figure 7****).** Secondary oxidation products (TBARS) 300 days after production are 18 µmol (kg oil)⁻¹ in F3, which is higher than in Intralipid (4µmol (kg oil)⁻¹), but clearly much lower than in OV (25 µmol (kg oil)⁻¹).

### Reduced total sterol load

F3 has the lowest amount of total sterols including phytosterols plus cholesterol, as measured by mass spectrometry (see **Figure 8** with 4 independent measurements of the indicated sterols in Intralipid, Omegaven, and F3). Notable, F3 also contains less stigmasterol than Intralipid, a phytosterol, which has been linked to liver inflammation.

### Example 8: Clinical areas of application of the novel lipid emulsion F3 with beneficial anti-inflammatory, liver protective, anti-diabetic and host defense strengthening actions

### Patients requiring short- and long-term Total Parenteral Nutrition (TPN)

TPN is a life-saving nutritional therapy under conditions where enteral feeding is contraindicated or insufficient. TPN is provided to millions of patients who are unable to orally ingest or digest and absorb the necessary daily amount of nutrients (partial or TPN). While parenteral nutrition is in many cases transient, lasting from days to weeks, thousands of patients every year in the United States alone require home-based long-term (>3 months) parenteral nutrition. In addition, in the United States half a million infants, including premature and low-birth-weight infants, depend on TPN. Indications for total or partial parenteral nutrition encompass a wide range of clinical conditions such as critically ill patients (trauma, surgery, sepsis, shock), patients on home parenteral nutrition because of chronic intestinal failure, cachectic cancer patients, patients with inflammatory bowel disease (Crohn's disease, ulcerative colitis), patients with gastrointestinal obstruction, high-output enterocutaneous fistula, or short-bowel syndrome, (mostly) geriatric patients with acute or chronic debilitating diseases who cannot meet nutritional requirements, and patients with intractable nausea and vomiting (hyperemesis gravidarum). Moreover, malnutrition (calories and/or protein related) is a common health care issue with a high prevalence among hospitalized patients (20-50%).

### Patients requiring short- and long-term Enteral Nutrition

Enteral and parenteral formulations are designed for subjects/patients who, given their particular condition, cannot meet nutritional needs through ordinary food consumption. The specifics of the patient's medical condition inform the application route for nutritional support. Parenteral lipid emulsions can be also administered via the enteral route (as part of a complete enteral formula) because the requirements for this route of administration with respect to sterility, osmolarity, and pH are much less stringent. The use of lipid emulsions in formulas of enteral application has the distinct advantage of minimizing the adverse effects of parenteral application (such as liver disease, metabolic disruption, immunosuppression and gut atrophy, see detailed review: Lucchinetti et al. Mol Nutr Food Res. 2021 Mar;65(5):e1901270.). Since enteral application of lipid emulsions results in complete absorption, uptake and distribution of the lipid emulsion and its compounds in the entire body, the same biological actions as observed with parenteral administration are expected to occur, specifically with regard to inflammation, metabolism and immune system.

### Patients with metabolic disease, specifically insulin resistance

The most prevalent metabolic disease in the world is glucose intolerance (also called pre-diabetes) with an estimated prevalence of 25-30% in Western populations. Patients with type I and II diabetes (up to 10%). All metabolic conditions where insulin resistance is of concern.

### Patients with reduced host defense

Many patients suffer from dysregulation of their immune system due to metabolic disorders, autoimmune disorders or infections.

### Patients with diseases of the liver

NASH (non-alcoholic steatohepatitis and cirrhosis) with a prevalence of between 30-50% in Western societies. Other liver diseases, which would benefit from the new lipid emulsion, are Alagille syndrome, alcohol- and medication-related liver disease, alpha-1 antitrypsin deficiency, autoimmune hepatitis, benign liver tumors, biliary atresia, cholestasis, Crigler-Najjar syndrome, galactosemia, Gilbert syndrome, hemochromatosis, hepatic encephalopathy, hepatitis A, hepatitis B, hepatitis C, hepatorenal syndrome, intrahepatic cholestasis of pregnancy, lysosomal acid lipase deficiency, liver cysts, liver cancer, newborn jaundice, primary biliary cholangitis, primary sclerosing cholangitis, progressive familial intrahepatic cholestasis, Reye Syndrome, Type I Glycogen Storage Disease, Wilson Disease and many more.

### Patients with systemic acute and/or chronic inflammation

This includes conditions with infectious diseases but also conditions with aseptic inflammation such as rheumatoid arthritis, autoimmune diseases such as systemic lupus erythematosus, multiple sclerosis, psoriasis, ankylosing spondylitis and many more.

### Example 9: Additional fields of application of the novel lipid emulsion F3

Lipid emulsions can be used as drug delivery systems (vehicle) for parenteral (injectable) or enteral administration of (lipophilic) drugs including biologicals and -more recently- for nucleic acid-based therapies (i.e. the use of nucleic acids and related compounds to alter gene expression for therapeutic purposes) and for vaccines. Additional applications of lipid emulsions in medicine are treatment of (lipophilic) drug overdose/poisoning (emulsion as detoxification) and prevention of ischemia-reperfusion injury (vital organ protection).

### Example 10: Drugs administered in lipid emulsions serving as vehicles

Applications of lipid emulsions in parenteral or enteral drug delivery have the distinct advantages of 1) reduction in pain, irritation, and thrombophlebitis 2) reduced toxicity 3) improved stability and solubility due to reduced degradation and 4) targeted drug delivery mainly to the liver. Examples of currently marketed drugs formulated with injectable lipid emulsions are diazepam, propofol, etomidate, alprostadil, dexamethasone, flurbiprofen, vitamins A, D, E, K, paclitaxel, cyclosporine. Many other drugs would be more stable in lipid emulsions, but specific formulations have not been created for the healthcare market so far. These include clarithromycin, phenobarbital, physostigmine, cinnarizine, chlorambucil, docetaxel, and many more.

In principle, all highly lipophilic drugs can be administered intravenously using lipid emulsions as safe vehicle.

### Example 11: RNA therapies using lipid emulsions as vehicles

This is an emerging field where lipid carrier systems are used as vehicle in gene therapies. RNA-lipid delivery systems have been and are used in clinical trials. For example siRNA-EphA2-DOPC targeting EPHA2 is used in advanced cancer (NCT 01591356), ALN-VSP02 targeting KSP and VEGF is used in solid cancer therapy (NCT 00882180), TKM-ApoB targeting ApoB is used for treatment of hypercholesterolemia (NCT 00927459).

### Example 12: Vaccine therapies using lipid emulsions as vehicle and adjuvants

Liposomes are ideal carriers in combined vaccines targeting several antigens and enhance the induction of antibodies and cell-mediated immunity. For example in production of a fivefold combined vaccine against hepatitis A and B, diphtheria, tetanus and influenza A/B, with good immunogenicity and excellent tolerance.

### Example 13: Additional applications of lipid emulsions as "rescue therapies" in medicine

Lipid emulsions can act as a "lipid sink" in patients intoxicated with lipophilic drugs such as local anesthetics, beta-blockers, neuroleptics, calcium blockers etc. In addition, they can be used for protection against ischemia-reperfusion injury in vital organs such as the heart, brain, liver, kidneys, and lungs.

### Discussion

The development of a novel lipid emulsion for TPN use was motivated by unsatisfactory clinical outcomes such as liver toxicity, diabetes-like metabolic conditions, and immunosuppression-associated infection risk in patients reliant on life-saving TPN using currently available lipid emulsions. The inventors' newly designed and engineered lipid emulsion contains high amounts of two shorter chain 18-carbon *n-3* fatty acids, namely α-linolenic acid (ALA) and stearidonic acid (SDA), and is optimized with regard to the recommended *n-6*/*n-3* ratio of 1:2.5. It is more resistant to oxidation and hydrolysis facilitating a longer shelf-life than 20/22-carbon *n-3* fatty acid-base lipid emulsions, and further contains a lower amount of the potentially toxic phytosterol stigmasterol than other plant oil-based lipid emulsions. The exclusive use of plant oils instead of algae, krill or fish oils for this emulsion, hence herein named TPN-F3 (VV), makes it more sustainable with regard to overfishing of seas and presents a lower risk of exposure to bioaccumulated marine toxins such as dioxin, mercury and radionuclides. The inventors' detailed comparisons with two commonly used lipid emulsions, namely soybean oil-based Intralipid and fish oil-based Omegaven, reveal that TPN-F3 possesses a unique combination of anti-inflammatory, insulin-sensitizing and immunity-enhancing properties, unmatched by currently available lipid emulsions. Specifically, we demonstrate that W-TPN releasing 18-carbon *n-3* fatty acids, mediates its beneficial actions by enhancing IL10-dependent insulin signaling and by boosting immunity.

During TPN, the immune system is continuously challenged with high amounts of bacterial toxins, namely endotoxin (LPS), leaking from the bowel into the portal system. While the total bacterial load in the gut is decreased due to lack of oral nutrients, gram-negative and invasive bacteria dominate the gut microbiome during TPN. Simultaneously, the immune system suffers from a catabolic metabolic state driven by insulin resistance. A recent study in mice suggests that insulin resistance plays a critical role in the occurrence of immune cell dysfunction as both insulin and T-cell receptor signaling converge at the same downstream kinase Akt. Using activated CD4⁺ T-cells lacking the insulin receptor, reduced proliferation and cytokine production, namely IFNγ, as well as impaired differentiation affecting Th1 and Th17 T-cells has been shown in mice. Intact insulin signaling appears to be instrumental for the metabolism of T-cells enabling a more efficacious immune response to pathogens. Another study in rats showed inadequate IL10 production in CD4⁺ T-cells in the absence of the insulin receptor. Insulin resistance also promotes "lazy" M2-like macrophages with impaired activation. Hence it is conceivable that W-TPN improved insulin signaling and thus reinforced immune cells as evidenced by the increased number of primed effector CD4⁺ T-cells (CD44^{low}/CD62L^{low}), which can acquire cytotoxic phenotypes or attract and modulate the activity of many innate immune cell. This boosted immunity may have ultimately helped eliminate the microinvasive bacterium *Akkermansia muciniphila* from the bowel mucosa. Of note, in IL-TPN, we observed a relative deficiency of IL10, specifically in liver tissue where IL6 concentrations were increased, which was not the case in W-TPN. Deficiency of IL10 is known to increase colonization of *Akkermansia muciniphila* with increased bacterial translocation and proinflammatory cytokine production including IL6. It also fosters the risk of colonization with other pathogens such as *Clostridium difficile* in the absence of immunosuppression. We have previously reported the significance of *Akkermansia muciniphila* blooming during TPN using the same mouse model. This bacterium has a growth advantage under nutrient deprivation such as prolonged fasting or TPN because it is capable of using mucins as a sole source of carbon and nitrogen. In line with the inventors' murine model, an increased abundance of *Akkermansia muciniphila* has been also demonstrated in the gut microbiome of TPN-fed infants. Importantly, despite the priming of immune cells in W-TPN mice, endotoxin contents in white adipose tissue were reduced and there was a marked reduction of inflammation as evidenced by a lower IL6/IL10 ratio and a decreased NFκB protein expression. Hence the inventors' results suggest that W-TPN, like an immunoadjuvant, has primed the immune system during TPN and enabled the body to eliminate potentially dangerous pathogens.

We did not observe increased production of IL6, IFNγ and TNFα in liver macrophages isolated from W-TPN mice when compared with IL-TPN. Thus, the increased IL6 and TNFα expressions in liver tissue of IL-TPN as opposed to W-TPN mice are likely due to activated hepatocytes. It further appears that macrophages, critical for both the innate nonspecific host defense and the adaptive specific immune response, were activated by IFNγ released from T-cells. IFNγ metabolically reprograms macrophages to sustain their viability and pro-inflammatory activity including ROS production by switching energy metabolism from OXPHOS to glycolysis, while IL10 reverses energy metabolism back to OXPHOS and inhibits activation of the NLRP3 inflammasome. This specific cytokine microenvironment may indeed underly the unique anti-inflammatory, but yet immunity-enhancing phenotype that we observed in W-TPN mice. In a clinical study with cancer patients comparing supplemental parenteral nutrition with olive-oil (n-9 fatty acids)- vs fish oil (long-chain *n-3* fatty acids)-based lipid emulsions, functions of the innate and adaptive immune system were higher in patients treated with olive oil, which is considered neural with regard to inflammation and immune stimulation. Fish oil-treated patients showed signs of immunosuppression evidenced by a reduced number of PMA-stimulated IFNγ-producing CD4⁺ T-cells and relatively high numbers of regulatory T-cells. Interestingly, in the inventors' own study, anti-endotoxin (LPS) IgG1 production was higher in mice treated with W-TPN, consistent with helper T-cell-mediated B-cell activation. These observations are in line with previous nutritional studies using 18-carbon ALA- and SDA-enriched plant oils. Patel et al. compared pups from pregnant Sprague-Dawley rats fed with SDA-enriched maternal diet with pubs from pregnant rats fed with control diet. The SDA-enriched diet resulted in higher B-cell function as measured by IgG1 production, higher numbers of activated helper T-cells, and splenocytes stimulated with endotoxin showed reduced IL6 and TNFα but increased IL10 production. Finally, a nutritional study in human volunteers with daily oral intake of 10 mL *Buglossoides Arvensis* oil rich in ALA and SDA for 4 weeks increased IL10 production in endotoxin-stimulated whole blood.

Hepatotoxicity is a major problem with TPN, and cases of severe steatohepatitis and portal fibrosis have been also reported in patients on fish oil-based TPN. Although we observed priming of immune cells in liver tissue, the actual number of leukocytes in the liver tissue was decreased during TPN when compared with chow-fed mice. In fact, W-TPN showed many liver protective features, namely a decrease in proinflammatory IL6 combined with an increase in anti-inflammatory IL10 when compared with standard lipid emulsions. IL10 is a protective factor against high fat diet-induced insulin resistance in the liver. Mice fed a high-fat diet and treated with a neutralizing anti-IL10 antibody showed increased expression of proinflammatory cytokines, mitochondria-dependent apoptotic signaling, and a disrupted insulin signaling with downregulation of IRS2 and reduced glycogen contents in the liver. LBP, a protein specifically produced by hepatocytes which detoxifies endotoxin, was upregulated exclusively in W-TPN. Likewise, SOCS3, a potent inhibitor of IL6 signaling, was upregulated exclusively in W-TPN. The increased abundance of LBP and SOCS3 inversely correlated with the IL6/IL10 ratio, suggesting a mechanistic contribution to lower inflammation in the liver. In addition, W-TPN upregulated anti-inflammatory PPARα similar to OV-TPN in liver tissue, but exclusively downregulated the proinflammatory and lipogenic PPARγ2. Finally, W-TPN increased liver tissue and more so pancreatic tissue concentrations of the anti-inflammatory helper T-cell cytokine IL4, which is known to beneficially affect β-cell function as well as lipid and glucose metabolism.

The immune-metabolic interplay during TPN is ultimately the result of the specific fatty acid species released from the administered lipid emulsion. While most TPN formulations have been shown to reduce the number of total T-cells in patients, TPN releasing primarily long-chain n-6 fatty acids further hampers T-cell function. The provision of specific fatty acids alters the composition of the phospholipid bilayers of cell membranes in T-cells, a process called "lipid remodeling", and thus changes the function of critical membrane-associated receptor proteins including the T-cell receptor. In the inventors' studies, provision of shorter chain 18-carbon *n-3* fatty acids primed CD4⁺ effector T-cell subpopulations expressing IFNγ and IL17, B-cells and macrophages. The detection of IL10 expression in these primed immune cells suggests T-cells, macrophages and possibly other cell types such as hepatocytes as likely sources of the elevated plasma and tissue IL10 concentrations. In fact, the occurrence of anti-IL10 effects in the presence of the isotype IgG control antibody, putatively via binding to Fc-receptors, pinpoints to immune cells as the main source of the elevated IL10. The different lipid emulsions used for TPN also evoked different profiles of biologically active lipid mediators. Lipid mediators, also called oxylipins, are the major source of fatty acid-induced biological actions in many tissues and cells including T-cells and macrophages. In the inventors' studies, the heatmap of the lipid mediators measured in liver tissue showed indeed striking differences among the three investigated TPNs. Liver tissue from W-TPN mice was devoid of the typical n-6 arachidonic acid-derived proinflammatory mediators, but also devoid of many of the long-chain n-3-derived anti-inflammatory mediators, except for a few EPA-derived mediators. Clearly, W-TPN elicited its own characteristic profile of oxylipins, which cannot be simply attributed to the bioconversion of shorter chain *n-3* fatty acids, namely ALA and SDA, to long-chain *n-3* fatty acids (EPA, DHA), since we did not observe increased formation of DHA-derived lipid mediators. In fact, a similar observation was reported in ALA-treated macrophages where 18-carbon lipid mediators, namely 9/13-HOTrEs and not EPA- or DHA-derived lipid mediators, were associated with reduced IL6 production in M1-polarized macrophages and increased IL10 production combined with enhanced phagocytosis in M2-polarized macrophages. Importantly, in the inventors' TPN mouse model, supplementation of IL-TPN with 9/13-HOTrEs mimicked many features of the immune-metabolic phenotype elicited by W-TPN. 9/13-HOTrEs have been previously shown to increase IL10 in mouse peritoneal macrophages, to inhibit NLRP3 inflammasome and to increase the survival in murine endotoxin and cecal ligation sepsis models. Recent studies also raise the possibility that activated lymphocytes and possibly other immune cells increase their own synthesis of lipid mediators, which could directly stimulate their activation and proliferation in an autocrine fashion.

While we report the beneficial biological actions of a novel TPN based on short-chain *n-3* fatty acids, additional studies are required to provide more mechanistic insights on 18-carbon *n-3* fatty acid-derived lipid mediators and their specific immune-metabolic actions in TPN. Also, it will be essential to show translatability of the observed beneficial immune-metabolic effects from the mouse model to patients.

In summary, the results of the inventors' study demonstrate that a novel lipid emulsion based on 18-carbon *n-3* fatty acids has remarkable anti-inflammatory, anti-diabetic and immunity-enhancing properties acting as "immunonutrition" during TPN. This unique profile, unmatched by currently available lipid emulsions, could be of particular benefit to vulnerable patients at risk of infection, sepsis patients with "immune paralysis" as well as cancer patients.

### Materials and methods

The investigation conforms to the Guide for the Care and Use of Laboratory Animals published by the US National Institutes of Health (NIH Publication No. 85-23, revised 1996) and adheres to the ARRIVE guidelines (https://www.nc3rs.org.uk/arrive-guidelines). The experimental protocol used in this investigation (AUP000002007) was approved by the University of Alberta Animal Policy and Welfare Committee.

### Preparation of the novel lipid emulsion TPN-F3 (F3 or VV)

1.2% lecithin (LIPOID E80, egg-derived phospholipids with 80% phosphatidylcholine, suitable for parenteral application, Lipoid GmbH, Ludwigshafen, Germany) was wetted in purified water (<20% of final volume; NANOpure Diamond Barnstead, Thermo Scientific Waltham MA, USA, or Endotoxin-free Ultra Pure Water, EMD Millipore Corp., Billerica MA, USA) in a water bath at 45 °C for 2 hrs. Dispersion was facilitated using a Polytron high-shear mixer (Polytron PT6000 drive unit, PT-DA 3012/2 TS dispersing aggregate, Kinematica AG, Malters, Switzerland) at 20'000 rpm for 60 sec. EDTA (EDTA disodium salt dihydrate, Carl Roth GmbH + Co. KG, Karlsruhe, Germany, ≥99%) 2.5 µM final concentration and glycerol 2.2% (Acros Organics, New Jersey, USA, 99+%) for isotonicity were added to the lecithin/water phase. 20% of the pre-mixed lipid phase consisting of 50% Ahiflower oil (Natures Crops International, Kensington PEI, Canada), 25% olive oil (LIPOID purified olive oil, Ph. Eur., Lipoid AG, Steinhausen, Switzerland) and 25% coconut oil (Bioriginal, Saskatoon SK, Canada) **(Table 4)** was added to the lecithin/water phase. 0.016% α-tocopherol (Sigma-Aldrich, St. Louis MO, USA, Type V, ~1000 IU/g) was supplemented and purified water was added to reach the final volume. The coarse emulsion was subsequently homogenized using a PL300 or HL60, respectively, high-pressure homogenizers (Dyhydromatics, Maynard MA, USA) equipped with a 75.1T reaction chamber and a 200.2L back-pressure module (only for PL300) to obtain the preferred droplet size of between 260-300 nm (six cycles at a pressure of 18 kpsi (PL300) to 22 kpsi (HL60)). pH was adjusted to >8.5 with 1 M NaOH until the ζ-potential was >|30| mV. Aliquots were filled into 50 mL glass bottles (Muller and Krempel AG, Bulach, Switzerland), the headspace was loaded with inert argon gas (PanGas AG, Dagmersellen, Switzerland, Argon 5.0), and the vial was crimped. Emulsions were autoclaved for 15 min at 121 °C at 2 bars (Systec DE-23, Systec GmbH, Linden, Germany). Absence of microbial growth (membrane-filtration method as per Ph Eur 2.6.1) and endotoxins (<0.1 IU/ml, gel-clot LAL gel-clot assay as per Ph Eur 2.6.14) was confirmed (Bioexam AG, Lucerne, Switzerland). The sterilized emulsions were further tested for droplet size by dynamic light scattering (Malvern Zetasizer 3000 HS A, Malvern Instruments, Malvern, United Kingdom), primary (mFOX assay) and secondary (TBARS assay) oxidative products, and non-esterified fatty acids were quantified by fluorescence detection after labelling with a fluorophore and separation by high-pressure liquid chromatography (FL-RP-HPLCREF). Cholesterol and phytosterols were determined using UPLC-MRM/MS and the composition of individual lipid emulsions was finally verified by gas chromatography (Agilent 6890 GC system). Absence of *in vitro* toxicity to T-cells was tested before each *in vivo* application.

Metabolic data in mice receiving total parenteral nutrition for 7 days (IL-TPN, OV-TPN, W-TPN) vs. conventional chow-fed mice with (IV-chow) or without saline infusion (chow).

Data are presented as mean (SD) or median (25^{th}, 75^{th} percentile). N=6.

GLP-1, glucagon-like peptide-1; HOMA-IR, Homeostatic Model Assessment for Insulin Resistance; IRβ, insulin receptor, beta subunit; IRS2, insulin receptor substrate 2; pY-IRS2, pan-tyrosine phosphorylated IRS2; OD, optical density; chow, non-instrumented C57BL/6J mice group-housed in static cages; IV-chow, chow-fed mice receiving heparinized physiological saline solution; IL-TPN, Intralipid-based total parenteral nutrition; OV-TPN, Omegaven-based total parenteral nutrition; VV-TPN, TPN-F3-based total parenteral nutrition.

Analysis of variance (ANOVA) was performed for the lipid infused groups only and differences were considered significant (boldface) if the overall P < 0.05. It was followed by multiple comparison procedures as appropriate. *, significantly decreased compared to OV-TPN and W-TPN; #, significantly increased compared to IL-TPN and OV-TPN; +, significantly increased as compared to OV-TPN; §, significantly increased compared to IL-TPN.

### TPN mouse model, treatment groups, formulation and dosing of TPN

The TPN mouse model used in this study has been previously described in detail (Lou et al. Molecular nutrition & food research 2021;65:e2000412.). Briefly, male C57BL/6 mice (22-25 g) were instrumented with a tunneled jugular vein catheter (JVC) and a magnetic vascular access button. A magnetic tether (VABM1T/25; Instech Laboratories Inc., Plymouth Meeting, PA, USA) mounted onto a swivel ensured freedom of movement and a programmable syringe pump (SAI InfusionTechnologies, Lake Villa IL, USA) was used to administer TPN. Mice were housed in single open conventional shoebox cages and maintained under controlled light conditions (12 h light/dark cycle) at a constant temperature of 21 °C and a relative humidity of 60% with free access to autoclaved water and regular chow diet (5L0D PicoLabLaboratory Rodent Diet; Canadian Lab Diets, Inc., Leduc County, AB, Canada). Mice were allowed to adapt for 4 days before experimentation and then randomly allocated to receive TPN with Intralipid (IL-TPN), TPN with Omegaven (OV-TPN), or TPN with TPN-F3 (W-TPN). Intralipid and Omegaven were purchased from Fresenius Kabi (Switzerland) AG (Kriens, Switzerland). On day 5, infusions were started. Mice allocated to TPN received continuous infusions of TPN solution starting at 0.25 mL hr⁻¹ (1st day of infusion, 6 mL per day), and subsequently increased to a maximum of 0.32 mL hr¹ (4th to 7th day of infusion, 7.7 mL per day). Mice infused with 0.9% saline with heparin (10 U mL⁻¹) at 6 mL per day and free access to water and chow served as controls (IV-chow). Age-matched non-instrumented C57BL/6 mice were housed for 7 days in conventional cages (3 mice per cage with free access to water and chow) and served as additional chow-fed controls (chow). Some mice with W-TPN were treated with 100 mg/24 hours of anti-mouse interleukin-10 (IL10) antibody (BioXCell #BE0049) added to the TPN mixture or its IgG1 isotype control antibody (BioXCell #BE0290). Some mice with IL-TPN were treated with 9/13-hydroxy-octadecatrienoic acids (5 ng/mL) added to the TPN mixture. Nutritional demands in terms of proteins were supplied as amino acid solution (4 kcal g⁻¹). Carbohydrates were supplied as glucose (3.4 kcal g⁻¹) and lipids as lipid emulsions (10 kcal g⁻¹). Mice with TPN received isocaloric (150 kcal per 100 mL), isonitrogenous TPN solutions containing either Intralipid, Omegaven, or TPN-F3.⁸ TPN provided 13% of the total calories from amino acids, 71% from glucose, and 16% from lipids with a non-protein energy to nitrogen ratio of 170. The values were calculated to satisfy the nutrient and energy requirements of mice weighing 24 g. For details on the TPN composition and dosing see **Table** 3. On day 8, tail blood glucose concentrations were measured using OneTouch VeriolQ (LifeScan Canada Ltd., Burnaby, BC Canada). Mice were disconnected from the infusion line, weighed, anesthetized with isoflurane, and euthanized by cervical dislocation prior to blood and tissue collections. Blood samples were obtained by cardiac puncture and immediately processed, while tissues were immediately frozen in liquid nitrogen and stored a -80 °C until analyses or further processed for immune cell isolation.

**Table 3: Composition of total parenteral nutrition (TPN) formulations used for in vivo mice experiments**

| Intralipid-based TPN (IL-TPN)/TPN-F3-based TPN (W-TPN) | |
|---|---|
| Nutrient source | amount per 100 mL |
| Amino acids (Aminoven 15%, Fresenius Kabi) | 32 mL (=4.8 g protein) |
| Glucose (70%) | 44.5 mL (31.2 g sugar) |
| Lipids (Intralipid 20%, Fresenius Kabi/TPN-F3 20%) | 12 mL (2.4 g fat) |
| Trace elements (Addaven, Fresenius Kabi) | 0.5 mL |
| Multivitamins (Cernevit, Baxter) | 0.1 mL |

| Omegaven-based TPN (OV-TPN) | |
|---|---|
| Nutrient source | amount per 100 mL |
| Amino acids (Aminoven 15%, Fresenius Kabi) | 32 mL (=4.8 g protein) |
| Glucose (80%) | 39 mL (31.2 g sugar) |
| Lipids (Omegaven 10%) | 24 mL (2.4 g fat) |
| Trace elements (Addaven, Fresenius Kabi) | 0.5 mL |
| Multivitamins (Cernevit, Baxter) | 0.1 mL |

The final formulations further contained sodium phosphate (13.4 mmol/L), electrolytes, and heparin (100 U/10 mL). The energy provided by all TPN formulations was similar (150 kcal/100 mL), 13% deriving from aminoacids, 16% from lipids, and 71% from carbohydrates (glucose). IL-TPN, Intralipid-based total parenteral nutrition; OV-TPN, Omegaven-based total parenteral nutrition; W-TPN, TPN-F3-based total parenteral nutrition.

### Hormone measurements, glycogen content, insulin receptor and (pY)insulin receptor substrate 2 (IRS2), and phosphoenolpyruvate carboxykinase (PEPCK) activity

Plasma insulin, glucagon, and glucagon-like peptide 1 (GLP-1) were measured from heparinized or EDTA-treated blood collected from cardiac puncture, using the following ELISA kits; Mercordia #10-1247-01 (insulin), Mercordia #10-1281-01 (glucagon), Crystal Chem #81508, GLP-1). Liver glycogen contents were measured from tissue powder using Sigma glycogen assay kit #MAK016. Total liver insulin receptor protein β subunit was determined using Insulin Receptor ELISA kit #KHR9111 (Thermo Fisher Scientific). Tyrosine phosphorylation of IRS2 and total-IRS2 were measured in liver lysate using a in-house ELISA assay. PEPCK activity of liver tissue was performed using a PEPCK activity kit (Abcam #ab239714) according to the manufacturer's recommendations.

### Cytokine profiling

Interferon-y (IFNγ) #DY485, interleukin-6 (IL6) #DY406, IL10 #DY417, and tumor necrosis factor-α (TNFα) #DY410) and interleukin-4 (IL4) #DY405 were measured from tissue powder using R&D DuoSet ELISA kits according to the manufacturer's instructions. 10 mg of powder were 2x rinsed in ice-cold PBS to remove residual blood before being homogenized in 100 mL ice-cold Lysis Buffer #6 (R&D Systems), using the Qiagen TissueLyser II (Qiagen) setup. The homogenates were left on ice for 15 min prior to centrifugation at 2,000g for 5 min. Supernatants were collected, and aliquoted for DC protein assay (Bio-Rad Laboratories) and DuoSet ELISA assays (R&D Systems) before storage at -80 °C. All cytokine measurements were normalized to sample protein concentration. Sample dilutions were performed when necessary, keeping the final urea concentration in all samples at 1 M prior to addition to the plate. Plasma IL10 was measured using R&D Quanbtikine ELISA kit #M1000B.

### Immunoblotting

Total tissue homogenates and nuclear/cytosolic fractions were prepared as previously reported (Lou et al. Molecular nutrition & food research 2021;65:e2000412.). Protein concentrations were determined by Bradford or DC protein assay (Bio-Rad Laboratories). Equal protein load was separated by SDS-PAGE and transferred to nitrocellulose membrane for probing with antibodies of interest. Immunoreactivity was visualized using ECL reagent (PerkinElmer) and quantified by ImageJ software. All immunoreactivity was normalized to vinculin or actin for total liver lysate or TATA-binding protein (TBP) for nuclear fractions, respectively. Glucokinase was detected by immunoblotting in both cytoplasmic and nuclear fractions and intensity of bands was normalized to vinculin, actin or TBP, respectively, for each band.

### Immune cell isolations

Immune cells from liver, spleen, mesenteric lymph, large and small bowel were isolated as previously described (Tsai et al. Cell metabolism 2018;28:922-34 e4). EasySep^{™} Mouse T Cell Isolation Kit (STEMCELL Technologies, Vancouver, BC, Canada) was used to negatively select T-cells by removing non-T-cells with biotinylated antibodies directed against non-T cells and streptavidin-coated magnetic particles. EasySep^{™} Mouse F4/80 Positive Selection Kit (STEMCELL Technologies) was used to isolate macrophages.

### Flow cytometry analysis

Cells obtained from each tissue (1-2 x 10⁶/sample) were stained with fluorophore-conjugated antibodies for innate immune cells and lymphocytes, run on an LSR Fortessa-SORP flow cytometer, and analyzed using FlowJo V10 software (BD, Ashland, Oregon, USA).

### Lipopolysaccharide (LPS) measurements and plasma anti-LPS IgG antibody concentrations

PyroGene^{™} Recombinant Factor C endotoxin detection fluorescence kit (Lonza #50-658U), with a minimum detection limit of 0.005 endotoxin (LPS) units (EU)/mL, was used to measure endotoxin levels in (epididymal) white adipose tissue. Mouse plasma anti-LPS IgG antibody concentrations against *E. coli* O111:B4 lipopolysaccharide were measured using a commercially available ELISA kit (Chondrex #6106).

### Microbiome analysis using 16S rRNA sequencing

Mucosal samples from the colon wall were collected at the end of the 7-day experimental period. DNA was extracted following the protocol of ZymoBIOMICS DNA Miniprep Kit (D4300, Zymo Research Corp; Irvine, CA, USA). The amplification of the V3-V4 hypervariable region was performed with the standard primers 341F (CCTACGGGNGGCWGCAG, SEQ ID NO: 1 and 805R (GACTACHVGGGTATCTAATCC, SEQ ID NO: 2). Libraries were prepared using the Metafast protocol, at Fasteris facilities (Genesupport/Fasteris SA, Plan-les-Ouates, Switzerland). 16S rRNA gene sequences were clustered into Operational Taxonomic Units (OTUs) and mapped to the SILVA Database. All data analysis was performed using the web-based tool MicrobiomeAnalyst.ca (https://www.microbiomeanalyst.ca/, Lucchinetti et al. Clin Nutrition ESPEN 2022).

### Quantitative profiling of lipid mediators using UHPLC-MS/MS

Lipid mediators were quantified using internal standards, calibrators, and quality controls, as previously described in detail (Hartling et al. Clinical chemistry and laboratory medicine 2021;59:1811-23.). Lipid mediators were extracted from liver tissue powders using methanol and solid phase extraction. Samples were evaporated under nitrogen and reconstituted for UHPLC-MS/MS injections.

### Statistics

Data are summarized as mean (SD) or median (25th, 75th percentile) depending on the underlying data distribution (normal vs skewed) for the indicated number of independent observations (N). Comparisons focused on TPN groups because of the study design aiming at a direct comparison between individual TPN groups. The significance of differences among the groups was determined by ANOVA followed by the Tukey method for posthoc analysis or by non-parametric methods (Kruskal-Wallis test followed by Dunn's test for posthoc analysis) depending on the underlying data distribution. For lipid mediator statistics, all concentrations were log-transformed and converted to z-scores for principal component analysis (PCA). Samples with concentrations below the detection limit were set to ½ limit of detection if the respective lipid mediator was detected in other samples of the same matrix. Unpaired *t*-test or Mann-Whitney U-tests, depending on the data distribution, was used to compare two groups if deemed. Differences were considered statistically significant if the overall *p* < 0.05 (two-sided). SigmaPlot (version 14.0; Systat Software Inc, San Jose, CA) was used for the analyses.

## Claims

1. **A lipid** emulsion for administration to a patient, wherein the lipid emulsion comprises an oily phase and an aqueous phase, wherein the oily phase of the lipid emulsion comprises:
- an omega-3 fatty acid component,
∘ wherein the mass of the omega-3 fatty acid component amounts to 25-35 % of the oily phase;
∘ wherein the omega-3 fatty acid component consists of one or more omega-3 fatty acids **characterized by** the presence of more than one carbon-carbon double bonds, wherein one carbon-carbon double bond is three atoms away from the terminal methyl group;
∘ and wherein the oily phase of the lipid emulsion comprises ≥ 5 % stearidonic acid;
∘ and wherein the oily phase of the lipid emulsion comprises ≥ 15 % α-linolenic acid (ALA);
- an omega-6 fatty acid component,
∘ wherein the mass of the omega-6 fatty acid component amounts to 10-15 % of the oily phase;
∘ wherein the omega-6 fatty acid component consists of one or more omega-6 fatty acids **characterized by** the presence of more than one carbon-carbon double bonds, wherein one carbon-carbon double bond six atoms away from the terminal methyl group;
- a monounsaturated fatty acid component,
∘ wherein the mass of the monounsaturated fatty acid component amounts to 18-30 % of the oily phase;
∘ wherein the monounsaturated fatty acid component consists of one or more fatty acids **characterized by** the presence of one carbon-carbon double bond;
- a saturated fatty acid component,
∘ wherein the mass of the saturated fatty acid component amounts to 20-35 % of the oily phase;
∘ wherein the saturated fatty acid component consists of one or more fatty acid **characterized by** no carbon-carbon double bond, but only carbon-carbon single bonds.

2. The lipid emulsion according to claim 1, wherein the ratio (m/m) of the omega-6 fatty acid component to the omega-3 fatty acid component ranges 1:3 to 1:2.

3. The lipid emulsion according to any one of the preceding claims, wherein the oily phase of the lipid emulsion comprises:
- 40-50 % PUFA (polyunsaturated fatty acid);
- 20-30 % MUFA (monounsaturated fatty acid); and
- 20-35 % SFA (saturated fatty acid).

4. The lipid emulsion according to any one of the preceding claims, wherein the oily phase of the lipid emulsion comprises:
- 5-15 % stearidonic acid;
- 20-30 % oleic acid;
- 5-15 % linoleic acid;
- 20-30 % α-linolenic acid; and
- 2-5 % γ-linolenic acid.

5. The lipid emulsion according to any one of the preceding claims, wherein the oily phase of the lipid emulsion comprises:
- 2-5 % of a short-chain fatty acid component selected from caproic, caprylic, and capric acid;
- 10-15 % lauric acid;
- 3-8 % myristic acid; and
- 5-12 % palmitic acid.

6. The lipid emulsion according to any one of the preceding claims, wherein the oily phase of the lipid emulsion comprises:
- 20-30 % olive oil;
- 20-30 % coconut oil; and
- 40-60 *% Buglossoides arvensis* oil.

7. The lipid emulsion according to any one of the preceding claims, wherein the lipid emulsion additionally comprises a stabilizer and/or an anti-oxidant selected from
- **EDTA;** and/or
- alpha tocopherol.

8. The lipid emulsion according to any one of the preceding claims 1 to 7, further comprising a pharmaceutical drug.

9. The lipid emulsion according to any one of the preceding claims 1 to 7 for use as a counteragent/detoxifyer in a treatment of intoxication caused by a lipophilic pharmaceutical drug or a lipophilic toxic compound.

10. The lipid emulsion according to any one of the preceding claims 1 to 8 for use in protection against ischemia-reperfusion injury of vital organs.

11. The lipid emulsion according to any one of the preceding claims 1 to 8 for use in prevention or treatment of diabetes mellitus type I or II.

12. The lipid emulsion according to any one of the preceding claims 1 to 8 for use in parenteral nutrition, wherein the parenteral nutrition is administered to a patient to whom one or several of the following indications apply:
- Patients requiring short- and long-term Total Parenteral Nutrition (TPN); and/or
- **TPN** Patients with metabolic disease; and/or
- TPN Patients with insulin resistance; and/or
- TPN Patients with a disease of the liver; and/or
- TPN Patients with systemic acute and/or chronic inflammation, and/or
- TPN Patients with reduced host defense, and/or
- Sepsis patients, and/or
- TPN Patients undergoing chemotherapy.

13. The lipid emulsion according to any one of the preceding claims 1 to 8 for use in enteral nutrition, wherein the enteral nutrition is administered to a patient to whom one or several of the following indications apply:
- Patients requiring short- or long-term enteral nutrition; and/or
- Patients with metabolic disease; and/or
- Patients with insulin resistance; and/or
- Patients with a disease of the liver; and/or
- Patients with systemic acute and/or chronic inflammation, and/or
- Patients with reduced host defense, and/or
- Sepsis patients, and/or
- Patients undergoing chemotherapy.

14. The lipid emulsion according to any one of the preceding claims 1 to 8, or the lipid emulsion for use according to claims 9 to 12, wherein the lipid emulsion is formulated for parenteral administration.

15. The lipid emulsion according to any one of the preceding claims 1 to 9, or the lipid emulsion for use according to claims 9 to 11 or 13, wherein the lipid emulsion is formulated for enteral or oral administration.

## Patentansprüche

1. Eine Lipidemulsion zur Verabreichung an einen Patienten, wobei die Lipidemulsion eine Ölphase und eine wässrige Phase umfasst, wobei die Ölphase der Lipidemulsion umfasst:
- eine Omega-3-Fettsäurekomponente,
∘ wobei die Masse der Omega-3-Fettsäurekomponente 25-35 % der Ölphase ausmacht;
∘ wobei die Omega-3-Fettsäurekomponente aus einer oder mehreren Omega-3-Fettsäuren besteht, die durch das Vorhandensein von mehr als einer Kohlenstoff-Kohlenstoff-Doppelbindung gekennzeichnet sind, wobei eine Kohlenstoff-Kohlenstoff-Doppelbindung drei Atome von der terminalen Methylgruppe entfernt ist;
∘ und wobei die Ölphase der Lipidemulsion ≥ 5 % Stearidonsäure enthält;
∘ und wobei die Ölphase der Lipidemulsion ≥ 15 % α-Linolensäure (ALA) enthält;
- eine Omega-6-Fettsäurekomponente,
∘ wobei die Masse der Omega-6-Fettsäurekomponente 10-15 % der Ölphase ausmacht;
∘ wobei die Omega-6-Fettsäurekomponente aus einer oder mehreren Omega-6-Fettsäuren besteht, die durch das Vorhandensein von mehr als einer Kohlenstoff-Kohlenstoff-Doppelbindung gekennzeichnet sind, wobei eine Kohlenstoff-Kohlenstoff-Doppelbindung sechs Atome von der terminalen Methylgruppe entfernt ist;
- eine einfach ungesättigte Fettsäurekomponente,
∘ wobei die Masse der einfach ungesättigten Fettsäurekomponente 18-30 % der Ölphase ausmacht;
∘ wobei die einfach ungesättigte Fettsäurekomponente aus einer oder mehreren Fettsäuren besteht, die durch das Vorhandensein einer Kohlenstoff-Kohlenstoff-Doppelbindung gekennzeichnet sind;
- eine gesättigte Fettsäurekomponente,
∘ wobei die Masse der gesättigten Fettsäurekomponente 20-35 % der Ölphase ausmacht;
∘ wobei die gesättigte Fettsäurekomponente aus einer oder mehreren Fettsäuren besteht, die **dadurch gekennzeichnet sind, dass** sie keine Kohlenstoff-Kohlenstoff-Doppelbindungen, sondern nur Kohlenstoff-Kohlenstoff-Einfachbindungen aufweisen.

2. Die Lipidemulsion nach Anspruch 1, wobei das Verhältnis (m/m) der Omega-6-Fettsäurekomponente zur Omega-3-Fettsäurekomponente im Bereich von 1:3 bis 1:2 liegt.

3. Die Lipidemulsion nach einem der vorstehenden Ansprüche, wobei die Ölphase der Lipidemulsion umfasst:
- 40-50 % PUFA (mehrfach ungesättigte Fettsäuren);
- 20-30 % MUFA (einfach ungesättigte Fettsäuren); und
- 20-35 % SFA (gesättigte Fettsäurem).

4. Die Lipidemulsion nach einem der vorstehenden Ansprüche, wobei die Ölphase der Lipidemulsion umfasst:
- 5-15 % Stearidonsäure;
- 20-30 % Ölsäure;
- 5-15 % Linolsäure;
- 20-30 % α-Linolensäure; und
- 2-5 % γ-Linolensäure.

5. Die Lipidemulsion nach einem der vorstehenden Ansprüche, wobei die Ölphase der Lipidemulsion umfasst:
- 2-5 % einer kurzkettigen Fettsäurekomponente, ausgewählt aus Capronsäure, Caprylsäure und Caprinsäure;
- 10-15 % Laurinsäure;
- 3-8 % Myristinsäure; und
- 5-12 % Palmitinsäure.

6. Die Lipidemulsion nach einem der vorstehenden Ansprüche, wobei die Ölphase der Lipidemulsion umfasst:
- 20-30 % Olivenöl;
- 20-30 % Kokosöl; and
- 40-60 % *Buglossoides arvensis* Öl.

7. Die Lipidemulsion nach einem der vorstehenden Ansprüche, wobei die Lipidemulsion zusätzlich einen Stabilisator und/oder ein Antioxidans umfasst, ausgewählt aus
- EDTA; und/oder
- alpha-Tocopherol.

8. Die Lipidemulsion nach einem der vorstehenden Ansprüche 1 bis 7, die ferner einen pharmazeutischen Wirkstoff umfasst.

9. Die Lipidemulsion nach einem der vorstehenden Ansprüche 1 bis 7 zur Verwendung als Gegenmittel/Entgiftungsmittel bei der Behandlung von Vergiftungen, die durch einen lipophilen pharmazeutischen Wirkstoff oder eine lipophile toxische Verbindung verursacht werden.

10. Die Lipidemulsion nach einem der vorstehenden Ansprüche 1 bis 8 zur Verwendung zum Schutz vor Ischämie-Reperfusionsschäden lebenswichtiger Organe.

11. Die Lipidemulsion nach einem der vorstehenden Ansprüche 1 bis 8 zur Verwendung bei der Vorbeugung oder Behandlung von Diabetes mellitus Typ I oder II.

12. Die Lipidemulsion nach einem der vorstehenden Ansprüche 1 bis 8 zur Verwendung in der parenteralen Ernährung, wobei die parenterale Ernährung einem Patienten verabreicht wird, auf den eine oder mehrere der folgenden Indikationen zutreffen:
- Patienten, die eine kurz- und langfristige totale parenterale Ernährung (TPN) benötigen; und/oder
- TPN-Patienten mit Stoffwechselerkrankungen; und/oder
- TPN-Patienten mit Insulinresistenz; und/oder
- TPN-Patienten mit einer Lebererkrankung; und/oder
- TPN-Patienten mit systemischen akuten und/oder chronischen Entzündungen; und/oder
- TPN-Patienten mit verminderter körpereigener Abwehr; und/oder
- Sepsis-Patienten; und/oder
- TPN-Patienten, die sich einer Chemotherapie unterziehen.

13. Die Lipidemulsion nach einem der vorstehenden Ansprüche 1 bis 8 zur Verwendung in der enteralen Ernährung, wobei die enterale Ernährung einem Patienten verabreicht wird, auf den eine oder mehrere der folgenden Indikationen zutreffen:
- Patienten, die eine kurz- und langfristige enterale Ernährung benötigen; und/oder
- Patienten mit Stoffwechselerkrankungen; und/oder
- Patienten mit Insulinresistenz; und/oder
- Patienten mit einer Lebererkrankung; und/oder
- Patienten mit systemischen akuten und/oder chronischen Entzündungen; und/oder
- Patienten mit verminderter körpereigener Abwehr; und/oder
- Sepsis-Patienten; und/oder
- Patienten, die sich einer Chemotherapie unterziehen.

14. Die Lipidemulsion nach einem der vorstehenden Ansprüche 1 bis 8 oder die Lipidemulsion zur Verwendung nach den Ansprüchen 9 bis 12, wobei die Lipidemulsion zur parenteralen Verabreichung formuliert ist.

15. Die Lipidemulsion nach einem der vorstehenden Ansprüche 1 bis 9 oder die Lipidemulsion zur Verwendung nach den Ansprüchen 9 bis 11 oder 13, wobei die Lipidemulsion zur enteralen oder oralen Verabreichung formuliert ist.

## Revendications

1. Émulsion lipidique pour l'administration à un patient, dans laquelle l'émulsion lipidique comprend une phase huileuse et une phase aqueuse, dans laquelle la phase huileuse de l'émulsion lipidique comprend :
- un composant d'acide gras oméga-3,
∘ dans laquelle la masse du composant d'acide gras oméga-3 représente 25 à 35 % de la phase huileuse ;
∘ dans laquelle le composant d'acide gras oméga-3 est constitué d'un ou plusieurs acides gras oméga-3 **caractérisés par** la présence de plus d'une double liaison carbone-carbone, dans laquelle une double liaison carbone-carbone est à trois atomes du groupe méthyle terminal ;
∘ et dans laquelle la phase huileuse de l'émulsion lipidique comprend ≥ 5 % d'acide stéaridonique ;
∘ et dans laquelle la phase huileuse de l'émulsion lipidique comprend ≥ 15 % d'acide α-linolénique (ALA) ;
- un composant d'acide gras oméga-6,
∘ dans laquelle la masse du composant d'acide gras oméga-6 représente 10 à 15 % de la phase huileuse ;
∘ dans laquelle le composant d'acide gras oméga-6 est constitué d'un ou plusieurs acides gras oméga-6 **caractérisés par** la présence de plus d'une double liaison carbone-carbone, dans laquelle une double liaison carbone-carbone est à six atomes du groupe méthyle terminal ;
- un composant d'acide gras monoinsaturé,
∘ dans laquelle la masse du composant d'acide gras monoinsaturé représente 18 à 30 % de la phase huileuse ;
∘ dans laquelle le composant d'acide gras monoinsaturé est constitué d'un ou plusieurs acides gras **caractérisés par** la présence d'une double liaison carbone-carbone ;
- un composant d'acide gras saturé,
∘ dans laquelle la masse du composant d'acide gras saturé représente 20 à 35 % de la phase huileuse ;
∘ dans laquelle le composant d'acide gras saturé est constitué d'un ou plusieurs acides gras **caractérisés par** aucune double liaison carbone-carbone, mais uniquement des liaisons simples carbone-carbone.

2. Émulsion lipidique selon la revendication 1, dans laquelle le rapport (m/m) du composant d'acide gras oméga-6 sur le composant d'acide gras oméga-3 va de 1/3 à 1/2.

3. Émulsion lipidique selon l'une quelconque des revendications précédentes, dans laquelle la phase huileuse de l'émulsion lipidique comprend :
- 40 à 50 % de PUFA (acide gras polyinsaturé) ;
- 20 à 30 % de MUFA (acide gras monoinsaturé) ; et
- 20 à 35 % de SFA (acide gras saturé).

4. Émulsion lipidique selon l'une quelconque des revendications précédentes, dans laquelle la phase huileuse de l'émulsion lipidique comprend :
- 5 à 15 % d'acide stéaridonique ;
- 20 à 30 % d'acide oléique ;
- 5 à 15 % d'acide linoléique ;
- 20 à 30 % d'acide α-linolénique ; et
- 2 à 5 % d'acide γ-linolénique.

5. Émulsion lipidique selon l'une quelconque des revendications précédentes, dans laquelle la phase huileuse de l'émulsion lipidique comprend :
- 2 à 5 % d'un composant d'acide gras à chaîne courte sélectionné parmi l'acide caproïque, caprylique et caprique ;
- 10 à 15 % d'acide laurique ;
- 3 à 8 % d'acide myristique ; et
- 5 à 12 % d'acide palmitique.

6. Émulsion lipidique selon l'une quelconque des revendications précédentes, dans laquelle la phase huileuse de l'émulsion lipidique comprend :
- 20 à 30 % d'huile d'olive ;
- 20 à 30 % d'huile de noix de coco ; et
- 40 à 60 % d'huile de *Buglossoides arvensis*.

7. Émulsion lipidique selon l'une quelconque des revendications précédentes, dans laquelle l'émulsion lipidique comprend en outre un stabilisant et/ou un antioxydant sélectionné parmi
- EDTA ; et/ou
- alpha tocophérol.

8. Émulsion lipidique selon l'une quelconque des revendications précédentes 1 à 7, comprenant en outre un médicament pharmaceutique.

9. Émulsion lipidique selon l'une quelconque des revendications précédentes 1 à 7 destinée à être utilisée en tant qu'antidote/détoxifiant dans un traitement d'intoxication provoquée par un médicament pharmaceutique lipophile ou un composé toxique lipophile.

10. Émulsion lipidique selon l'une quelconque des revendications précédentes 1 à 8 destinée à être utilisée dans la protection d'une lésion d'ischémie-reperfusion d'organes vitaux.

11. Émulsion lipidique selon l'une quelconque des revendications précédentes 1 à 8 destinée à être utilisée dans la prévention ou le traitement du diabète sucré de type I ou II.

12. Émulsion lipidique selon l'une quelconque des revendications précédentes 1 à 8 destinée à être utilisée dans la nutrition parentérale, dans laquelle la nutrition parentérale est administrée à un patient auquel une ou plusieurs des indications suivantes s'appliquent :
- des patients nécessitant une nutrition parentérale totale (TPN) à court et long terme ; et/ou
- des patients TPN avec une maladie métabolique ; et/ou
- des patients TPN avec une résistance à l'insuline ; et/ou
- des patients TPN avec une maladie du foie ; et/ou
- des patients TPN avec une inflammation aigüe et/ou chronique systémique, et/ou
- des patients TPN avec une défense de l'hôte réduite, et/ou
- des patients atteints de sepsie, et/ou
- des patients TPN subissant une chimiothérapie.

13. Émulsion lipidique selon l'une quelconque des revendications précédentes 1 à 8 destinée à être utilisée dans la nutrition entérale, dans laquelle la nutrition entérale est administrée à un patient auquel une ou plusieurs des indications suivantes s'appliquent :
- des patients nécessitant une nutrition entérale à court ou long terme ; et/ou
- des patients avec une maladie métabolique ; et/ou
- des patients avec une résistance à l'insuline ; et/ou
- des patients avec une maladie du foie ; et/ou
- des patients avec une inflammation aigüe et/ou chronique systémique, et/ou
- des patients avec une défense de l'hôte réduite, et/ou
- des patients atteints de sepsie, et/ou
- des patients subissant une chimiothérapie.

14. Émulsion lipidique selon l'une quelconque des revendications précédentes 1 à 8 ou émulsion lipidique destinée à être utilisée selon les revendications 9 à 12, dans laquelle l'émulsion lipidique est formulée pour l'administration par voie parentérale.

15. Émulsion lipidique selon l'une quelconque des revendications précédentes 1 à 9 ou émulsion lipidique destinée à être utilisée selon les revendications 9 à 11 ou 13, dans laquelle l'émulsion lipidique est formulée pour l'administration par voie entérale ou orale.
